(19) Europäisches Patentamt
European Patent Office
Office européen des brevets

(11) Publication number: **0 365 763 A1**

(12) **EUROPEAN PATENT APPLICATION**

(21) Application number: **89113994.1**

(22) Date of filing: **28.07.89**

(51) Int. Cl.⁵: **C07D 239/84 , C07D 239/88 , C07D 239/90 , C07D 239/91 , C07D 239/93 , C07D 239/95 , C07D 307/89 , C07D 401/12 , A61K 31/505 , C07D 405/12 , C07D 417/12**

(30) Priority: **30.09.88 US 251765**

(43) Date of publication of application: **02.05.90 Bulletin 90/18**

(84) Designated Contracting States: **AT BE CH DE ES FR GB GR IT LI LU NL SE**

(71) Applicant: **AGOURON PHARMACEUTICALS**
**P.O. Box 12209 11025 Torrey Pines Road**
**LaJolla California 92037(US)**

(72) Inventor: **Jones, Terence R.**
**1962 Missouri Street**
**San Diego, CA 92109(US)**
Inventor: **Varney, Michael D.**
**7236 Mimosa Drive**
**Carlsbad, CA 92009(US)**
Inventor: **Webber, Stephen E.**
**3884 Mt. Abraham Avenue**
**San Diego, CA 92111(US)**
Inventor: **Appelt, Krysztof**
**664 Stratford**
**Encinitas, CA 92024(US)**
Inventor: **Marzoni, Gifford**
**2656 Pikake Street**
**San Diego, CA 92154(US)**

(74) Representative: **Patentanwälte Grünecker, Kinkeldey, Stockmair & Partner**
**Maximilianstrasse 58**
**D-8000 München 22(DE)**

(54) **Antiproliferative cyclic compounds.**

(57) A compound of formula (I) capable of inhibiting thymidylate synthase, a method for making such and a therapeutic process utilizing the compound of formula (I).

$$\text{(I)} \qquad R_L-CH_2-\overset{\displaystyle R^2}{\overset{|}{N}}-(RiSys)$$

EP 0 365 763 A1

# ANTIPROLIFERATIVE CYCLIC COMPOUNDS

## FIELD OF THE INVENTION

The present invention relates to certain cyclic compounds which inhibit the enzyme thymidylate synthase ("TS"), to pharmaceutical compositions containing these cyclic compounds, and to the use of these compounds to inhibit TS, including all effects derived from the inhibition of TS. Effects derived from the inhibition of TS include the inhibition of the growth and proliferation of the cells of higher organisms and of microorganisms, such as yeast and fungi. Such effects include antitumor activity. A process for the preparation of the cyclic compounds of the invention is also disclosed.

## BACKGROUND OF THE INVENTION

The large class of antiproliferative agents includes antimetabolite compounds. A particular subclass of antimetabolites known as antifolates or "antifols" are antagonists of the vitamin. folic acid. Typically, antifolates closely resemble the structure of folic acid, including the characteristic p-benzoyl glutamate moiety of folic acid. Because the glutamate moiety of folic acid takes on a double negative charge at physiological pH, this compound and its analogues cannot passively diffuse into a cell and must have an active, energy-driven transport system to cross the cell membrane and exert a metabolic effect.

The earliest antifolates were aminopterin and methotrexate ("MTX"). MTX has been widely used in the treatment of human neoplastic diseases, such as malignant diseases. The cytotoxic action of MTX is generally ascribed to its inhibition of the enzyme dihydrofolate reductase (DHFR), a key enzyme which maintains the pools of one-carbon carrying tetrahydrofolates. One of the known causes of resistance to MTX, which has the glutamate moiety characteristically found in folic acid analogues, is reduced transport across the cell membrane.

Two more recent inhibitors of DHFR, piritrexim and trimetrexate, both of which lack the glutamate moiety, have been developed and have entered clinical trials. These two agents penetrate the cell wall by passive diffusion and, thus, can affect both normal tumor cells and those resistant because of a transport defect. These compounds have been found active even against tumor cells having an MTX transport defect. Because they are more soluble in organic solvents and in lipids than MTX, they have been termed lipophilic DHFR inhibitors. Additionally, trimetrexate may be active against opportunistic infections which occur in patients infected with HIV (human immunodeficiency virus, AIDS).

While the enzyme DHFR is a valid target for an antifolate, it appears that thymidylate synthase ("TS") is even better. TS is an enzyme that catalyzes the C-methylation reaction of 2′-deoxyuridylate (dUMP) to provide 2′-deoxythymidylate (dTMP). This one-carbon transfer reaction is critical to cell division because it provides the sole de novo source of dTMP, an essential building block for DNA synthesis. Hence, TS has long been considered an important target enzyme in the design and synthesis of antitumor agents, and a number of folate analogues have been synthesized and studied for their ability to inhibit TS. See, for example, Brixner et al., Folate Analogues as Inhibitors of Thymidylate Synthase, J. Med. Chem. 30, 675 (1987); Jones et al., Quinazoline Antifolates Inhibiting Thymidylate Synthase: Benzoyl Ring Modifications, J. Med. Chem., 29, 468 (1986); Jones et al., Quinazoline Antifolates Inhibiting Thymidylate Synthase: Variation of the Amino Acid, J. Med. Chem., 29, 1114 (1986); and Jones et al., Quinazoline Antifolates Inhibiting Thymidylate Synthase: Variation of the $N^{10}$ Substituent, J. Med. Chem. 28, 1468 (1985).

Recently, a specific tight-binding inhibitor of TS, 10-propargyl-5,8-dideazafolic acid (I: R = $NH_2$ below) was described. In clinical trials, this compound has shown activity against ovarian, liver and breast cancer. However, troublesome side effects, such as hepatic toxicity and dose-limiting renal toxicity, have sometimes been observed. The insoluble nature of the compound has been thought to be the cause of these side effects. Accordingly, by removing the amino group, a more soluble, more potent, less organ-toxic compound has been produced (II: R = H below). Jones et al., Synthesis and Some Biological Properties of 2-Desamino-10-Propargyl-5,8-Dideazafolic Acid and Related $N^{10}$ Analogues, Proc. Am. Assoc. Cancer. Res. 28, 276 (1987) and, Jackman et al., 2-Desamino-10-Propargyl-5,8-Dideazafolic Acid (Desamino CB3717) A Thymidylate Synthase (TS) Inhibitor Devoid of Renal and Hepatic Toxicities in Mice, Proc. Am. Assoc. Cancer Res., 28, 271 (1987). In an extension of this work, it was found that certain 2-substituted-2-desamino quinazolines exhibited more favorable pharmacological properties, particularly the 2-methyl compound (III: R

= $CH_3$ below). Newell et al., Pharmacokinetic and Toxicity Studies with $C_2$-Desamino-$C_2$-Substituted Analogues of the Antifolate $N_{10}$-Propargyl-5,8-Dideazafolic Acid (CB3717), Proc. Am. Assoc. Cancer Res., 29, 286 (1988); Hughes et al., Thymidylate Synthase (TS) Inhibitory and Cytotoxid Activity of a Series of $C_2$ Substituted-5,8-deazafolates, Proc. Am. Assoc. Cancer Res., 29, 286 (1988); and Jackman et al., Proc. Am. Assoc. Cancer Res., 29, 287, (1988).

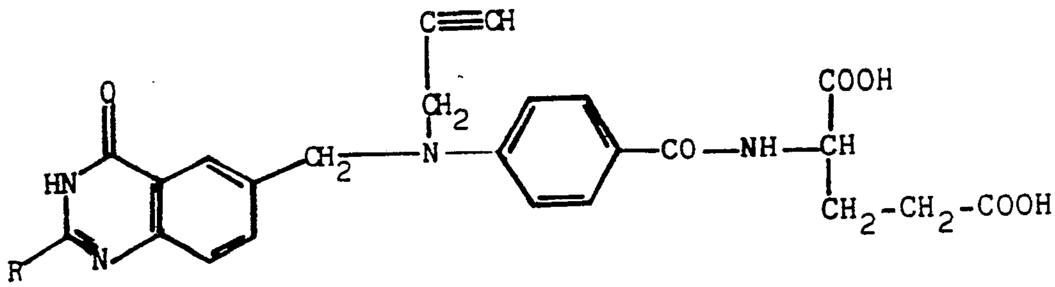

I:R = $NH_2$
II:R = H
III:R = $CH_3$

A well-known aspect of folate biochemistry is the phenomenon of polyglutamation. Once folic acid and its analogues, such as MTX and the above compounds I, II and III, have been transported inside the cell, these molecules, which already carry a single glutamate moiety, gain additional gamma-linked glutamate moieties. The polyglutamate metabolites, having extra appended glutamate moieties, cannot then efflux the cell and, moreover, bind even more tightly to certain folate matabolizing enzymes than the original folic acid or analogue parent compound. This enhanced binding is negligible in the case of the DHFR enzyme, but can be considerable in the case of TS. McGuire et al., Mechanism of Action of 5,8-Dideazaisofolic Acid and Other Quinazoline Antifols in Human Colon Carcinoma Cells, Cancer Res., 47, 5975 (1987), and Cheng et al., Activity of the New Antifolate $N^{10}$-Propargyl-5,8-dideazafolate and Its Polyglutamates against Human Dihydrofolate Reductase, Human Thymidylate Synthetase, and KB Cells Containing Different Levels of Dihydrofolate Reductase, Cancer Res., 45, 598 (1985).

For example, it has been found that the tetraglutamate of 10-propargyl-5,8-dideazafolic acid (I: R = $NH_2$) binds 119-fold more tightly to murine L1210 TS than the monoglutamate parent compound itself. E. Sikora et al., Chemistry and Biology of Pteridines 1986, 675 (B. Cooper and V. Whitehead ed. 1986). The formation of such a tightly-binding polyglutamate moiety can only take place if the compound has at least one glutamate moiety in the first place. Moreover, the first glutamate moiety in 10-propargyl-5,8-dideazafolic acid (I: R = $NH_2$) seems to be particularly important because the analogue of this compound completely lacking a glutamate moiety has been found to be 84-fold less active that the parent compound as a TS inhibitor. Jones et al., Quinazoline Antifolates Inhibiting Thymidylate Synthase: Variation of the Amino Acid, J. Med. Chem., 29, 1114 (1986). Thus, complete removal of the glutamate moiety from the tetraglutamate compound leads to a loss of about 4 orders of magnitude (84 x 119) of binding to TS.

## DESCRIPTION OF THE INVENTION

It has been found, according to the present invention, that certain cyclic compounds which lack the p-benzoyl glutamate moiety characteristically found on TS inhibitors are unexpectedly capable of inhibiting TS by binding to this enzyme.

One aspect of the present invention is a compound, capable of inhibiting thymidylate synthase having the formula (I):

(I)

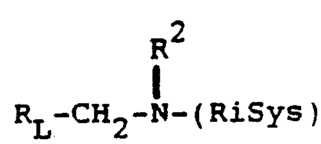

wherein, in said formula (I):

$R_L$ is (i) a substituted or unsubstituted heterocyclic ring system or (ii) a group of the formula (II):

(II) 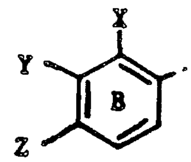

wherein, in said formula (II),

(a) X, Y and Z are independently hydrogen or separate substituents, with the proviso that at least one of X, Y and Z is not hydrogen,

(b) X and Y together comprise the nonmetallic atoms necessary to complete a substituted or unsubstituted homocyclic or heterocyclic ring, thereby forming a bicyclic ring system with phenyl group B, and Z is hydrogen or a separate substituent,

(c) Y and Z together comprise the nonmetallic atoms necessary to complete a substituted or unsubstituted, monocyclic or bicyclic, homocyclic or heterocyclic ring system, thereby forming a bicyclic or tricyclic ring system with phenyl group B, and X is hydrogen or a separate substituent, with the proviso that when a 6-quinazolino group is so formed and X is a methyl group, the ring system does not have $-NH_2$ groups in both the 2-and 4-positions, or

(d) X and Y together comprise the nonmetallic atoms necessary to complete a substituted or unsubstituted homocyclic or heterocyclic ring, and Y and Z together comprise the nonmetallic atoms necessary to complete a substituted or unsubstituted, monocyclic or bicyclic, homocyclic or heterocyclic ring system, thereby forming a peri-fused tricyclic or tetracyclic ring system with phenyl group B; with the proviso that $R_L$ is not a pteridine moiety, and

where X is a separate substituent, X is selected from the group consisting of lower alkyl, lower haloalkyl, lower hydroxyalkyl, lower aminoalkyl, hydroxy, lower alkoxy, lower acyloxy, mercapto, lower alkylthio, lower alkylsulfinyl, lower alkylsulfonyl, lower acylthio, amino, lower alkylamino, lower acylamino, lower alkoxy carbonyl, lower acyl, carbamoyl, N-lower alkylcarbamoyl, halo, cyano, nitro and azido groups,

where Y is a separate substituent, Y is selected from the group consisting of alkyl, homoaryl, heteroaryl, hydroxyalkyl, aminoalkyl, alkylaminoalkyl, haloalkyl, hydroxy, alkoxy, aryloxy, acyloxy, aroyloxy mercapto, alkylthio, alkylsulfinyl, alkylsulfonyl, arylthio, arylsulfinyl, arysulfonyl, pentfluorothio, acylthio, aroylthio, sulfamoyl, N-alkylsulfamoyl, N-arylsulfamoyl, sulfinamoyl, N-sulfinamoyl, N-arylsulfinamoyl, N-hydroxysulfamyl, amino, alkylamino, arylamino, acylamino, aroylamino, sulfonamido, guanidino, hydrazino, acylhydrazino, alkoxycarbonyl, carbamoyl, N-alklycarbamoyl, N-arylcarbamoyl, amidino, hydrazinocarbonyl, hydroxyaminocarbonyl, alkoxyaminocarbonyl, acyl, aroyl, halo, cyano, nitro and azido groups, with alkyl and acyl groups containing from 1 to 6 carbon atoms, and

where Z is a separate substituent, Z is selected from the group consisting of

alkyl, homoaryl, heteroaryl, hydroxyalkyl, aminoalkyl, alkylaminoalkyl, haloalkyl, hydroxy, alkoxy, aryloxy, acyloxy, aroyloxy mercapto, alkylthio, alkylsulfinyl, alkylsulfonyl, arylthio, arylsulfinyl, arylsulfonyl, pentfluorothio, acylthio, aroylthio, sulfamoyl, N-alkylsulfamoyl, N-arylsulfamoyl, sulfinamoyl, N-sulfinamoyl, N-arylsulfinamoyl, N-hydroxysulfamyl, amino, alkylamino, arylamino, acylamino, aroylamino, sulfaonamido, guanidino, hydrazino, acylhydrazino, alkoxycarbonyl, carbamoyl, N-alkylcarbamoyl, N-arylcarbamoyl, amidino, hydrazinocarbonyl, hydroxyaminocarbonyl, alkoxyaminocarbonyl, acyl, aroyl, halo, cyano, nitro and azido groups, with alkyl and acyl groups containing from 1 to 6 carbon atoms.

$R^2$ is hydrogen; substituted or unsubstituted alkyl having from 1 to 6 carbon atoms; substituted or unsubstituted alkenyl having from 1 to 6 carbon atoms; substituted or unsubstituted alkynyl having from 1 to 6 carbon atoms; aroylalkyl having from 1 to 10 carbon atoms; acyl having from 1 to 6 carbon atoms; $-OR'$ or $-N(R'R'')$ where $R'$ and $R''$ are independently hydrogen, alkyl, aryl, heteroaryl, acyl, aroyl, sulfonyl, sulfinyl or heteroaroyl groups; or $-SO_2-R'''$ or $-SO-R'''$ where $R'''$ is substituted or unsubstituted alkyl, aryl, heteroaryl or alkenyl; and

(RiSys) is a substituted or unsubstituted homocyclic or heterocyclic ring system having from 3 to 10 ring atoms,

with the following further proviso:

(i) when (RiSys) is substituted phenyl, (RiSys) is not substituted in the para-position with any of the following groups: $-SO_2$-glutamate; $-SO_2$-aspartate; or $-CO-NHR_b$ where $R_b$ is such that $NH_2-R_b$ is an amino acid, a poly(amino acid), a lower alkyl ester or an amino acid, or a lower alkyl ester of a poly(amino acid) and

(ii) when $R_L$ is 2-amino-3, 4-dihydro-4-oxo-6-quinazolinyl and (RiSys) is substituted phenyl, (RiSys) is not substituted in the para-position with -COOH, $-(CH_2)_3-COOCH_3$, $-(CH_2)_3-COOH$, or $-COOC_2H_5$.

As used herein, the term "a compound capable of inhibiting thymidylate synthase" denotes a compound with a TS inhibition constant $K_i$ of less than or equal to about $10^{-4}$M. The compounds of the invention preferably have $K_i$ values in the range of less than about $10^{-5}$M, more preferably less than about $10^{-6}$M, even more preferably less than about $10^{-9}$, and most preferably in the range from about $10^{-12}$ to about $10^{-4}$.

Further, as used herein, the range or number of carbon atoms in the term "a substituted group having a certain range or number of carbon atoms" denotes the range or number of carbon atoms in the base group, that is, the group in unsubstituted form.

When X in formula (II) is a separate substituent, X may be, for example, lower alkyl, lower haloalkyl, lower hydroxyalkyl, lower aminoalkyl, hydroxy, lower alkoxy, lower acyloxy, mercapto, lower alkylthio, lower alkylsulfinyl, lower alkylsulfonyl, lower acylthio, amino, lower alkylamino, lower acylamino, lower alkoxycarbonyl, lower acyl, carbamoyl, N-lower alkylcarbamoyl, halo, cyano, nitro or azido.

When Y in formula (II) is a separate substituent, Y may be, for example, alkyl, homoaryl, heteroaryl, hydroxyalkyl, aminoalkyl, alkylaminoalkyl, haloalkyl, hydroxy, alkoxy, aryloxy, acyloxy, aroyloxy, mercapto, alkylthio, alkylsulfinyl, alkylsulfonyl, arylthio, arylsulfinyl, arylsulfonyl, pentafluorothio, acylthio, aroylthio, sulfamoyl, N-alkylsulfamoyl, N-arylsulfamoyl, sulfinamoyl, N-alkylsulfinamoyl, N-arylsulfinamoyl, N-hydroxysulfamyl, amino, alkylamino, arylamino, acylamilno, aroylamino, sulfonamido, guanidino, hydrazino, acylhydrazino, alkoxycarbonyl, carbamoyl, N-alkylcarbamoyl, N-arylcarbamoyl, amidino, hydrazinocarbonyl, hydroxyaminocarbonyl, alkoxyaminocarbonyl, acyl, aroyl, halo, cyano, nitro or azido, with alkyl and acyl groups preferably containing from 1 to 6 carbon atoms.

When Z in formula (II) is a separate substituent, Z may independently be the same as any of the examples given above for Y.

$R_L$ in formula (I) may, for example, be a group of the formula (II) where X, Y and Z are independently hydrogen or the nonmetallic atoms necessary to complete a substituted or unsubstituted 5- or 6-membered heterocyclic ring. Preferred $R_L$ groups may include those of formula (II) having the structures

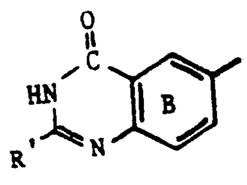

or

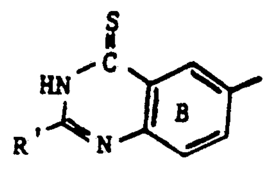

wherein $R^1$ may be alkyl such as methyl, ethyl, propyl, isopropyl, isobutyl, tert-butyl, pentyl and hexyl; cycloalkyl such cyclopropyl, cyclopentyl and cyclohexyl; alkenyl such as prop-2-enyl, but-2-enyl, but-3-enyl, 2-methylprop-2-enyl, hex-2-enyl, hex-5-enyl and 2,3-dimethylbut-2-enyl; alkynyl such as prop-2-ynyl, but-2-ynyl, but-3-ynyl, pent-2-ynyl, 3-methylpent-4-ynyl, hex-2-ynyl and hex-5-ynyl; alkoxy such as methoxy, ethoxy, propoxy, isopropoxy and hexyloxy; alkylthio such as methylthio, isopropylthio and hexylthio; aryl such as phenyl and heterocyclyl; aryloxy such as phenoxy and tolyloxy; arylalkyl such as tolyl, benzyl, alpha-methylbenzyl and phenethyl; halogeno such as fluoro, chloro, bromo and iodo; hydroxy; amino, mercapto; pyridylthio; pyrimidinylthio; substituted alkyl such as fluoromethyl, difluoromethyl, trifluoromethyl, 2-fluoroethyl, 3-fluoropropyl, chloromethyl, dichloromethyl, hydroxymethyl, 2-hydroxyethyl, 3-hydroxypropyl, aminomethyl, 3-aminopropyl, pyrid-2-ylthiomethyl, pyrimidin-2-ylthiomethyl, methoxymethyl, isopropoxymethyl, 3-methoxypropyl, acetoxymethyl, propionyloxymethyl, methylthiomethyl, 3-methylthiopropyl, propylthiomethyl, methylaminomethyl, propylaminomethyl, methylaminopropyl, dimethylaminomethyl, diethylaminomethyl, ethylmethylaminomethyl, 3-dimethylaminopropyl, acetamidomethyl, 3-acetamidopropyl, propionamidomethyl, benzoyloxymethyl and benzamidomethyl; or substituted alkoxy such as 2-hydroxyethoxy, 4-hydroxybutoxy, 3-hydroxy-2-methylpropoxy, 2-methoxyethoxy, 3-methoxypropoxy and 2-ethoxyethoxy. Most preferably, $R^1$ is alkyl such as methyl, or alkoxy such as methoxy.

Other $R_L$ groups of formula (II) include those having the following structures:

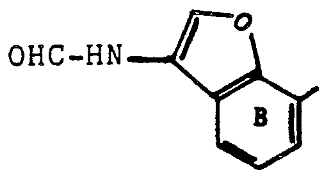

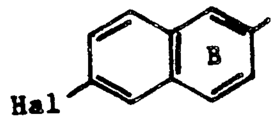

where Hal- is a halogen atom. Compounds having these $R_L$ groups may preferably also have a prop-2-ynyl group in the $R^2$ position and a 3-trifluoromethyl- or 4-nitrophenyl group as (RiSys), as described below.

$R_L$ may also be a substituted or unsubstituted heterocyclic ring system, such as thiophene, pyridine, thienopyridine, thienothiazole, triazinoindolizone and pyridopyridine. A preferred example of such compounds is a substituted or unsubstituted thienopyrimidine ring.

$R_L$ does not include pteridine moieties, such as those pteridine moieties contained in an aromatic pteridine or tetrahydropteridine group.

$R^2$ in formula (I) may be hydrogen; alkyl preferably having from 1 to 6 carbon atoms, more preferably from 1 to 3 carbon atoms, such as methyl, ethyl, propyl and isopropyl; alkenyl, preferably having from 1 to 6 carbon atoms, more preferably from 1 to 3 carbon atoms, such as prop-2-enyl; alkynyl, preferably having from 1 to 6 carbon atoms, more preferably from 1 to 3 carbon atoms, such as prop-2-ynyl; substituted alkyl having from 1 to 6 carbon atoms such as hydroxyalkyl (e.g., 2-hydroxyethyl and 3-hydroxypropyl), alkoxyalkyl (e.g., 2-methoxyethyl, 2-ethoxyethyl, 3-methoxypropyl and 2-methoxypropyl), mercaptoalkyl (e.g, 2-mercaptoethyl and 3-mercaptopropyl), alkylthioalkyl (e.g., 2-methylthioethyl, 3-methylthiopropyl and 2-ethylthioethyl), halogenoalkyl (e.g., 2-fluoroethyl, 2-chloroethyl, 2-bromoethyl, 3-fluoropropyl and 3-chloropropyl), cyanoalkyl (e.g., cyanomethyl, 2-cyanoethyl and 3-cyanopropyl), aminoalkyl (e.g., 2-aminoethyl, 3-aminopropyl and 3-amino-2-methylpropyl), alkylaminoalkyl (e.g., 2-methylaminoethyl, 2-ethylaminoethyl and 3-methylaminopropyl), dialkylaminoalkyl (e.g, 2-dimethylaminoethyl, 2-diethylaminoethyl and 3-dimethylaminopropyl), and alkanoylalkyl (e.g., acetonyl, 2-acetylethyl, propionylmethyl, 2-propionylethyl, 3-acetylpropyl, 4-acetylbutyl), carboxyalkyl (e.g., carboxymethyl, 2-carboxyethyl), carbamoylalkyl (e.g. carbamoylmethyl) or alkanoyl (e.g. acetyl, propionyl and butyryl); substituted alkenyl having up to six carbon atoms such as hydroxyalkenyl, alkoxyalkenyl, mercaptoalkenyl, alkylthioalkenyl, halogenoalkenyl, cyanoalkenyl, aminoalkenyl, alkylaminoalkenyl, dialkylaminoalkenyl, alkanoylalkenyl, carboxyalkenyl and carbamoylalkenyl; or substituted alkynyl having up to six carbon atoms such as hydroxyalkynyl, halogenoalkynyl, cyanoalkynyl, aminoalkynyl, alkylaminoalkynyl, alkanoylalkynyl, carboxyalkynyl and carbamoylalkynyl; or aroylalkyl having up to 10 carbon atoms such as phenacyl or 2-benzoylethyl; acyl having from 1 to 6 carbon atoms; $-OR'$ or $-N(R'R'')$ where $R'$ and $R''$ are independently hydrogen, alkyl, aryl, heteroaryl, acyl, aroyl, sulfonyl, sulfinyl or heteroaroyl groups; or $-SO_2-R'''$ or $-SO-R'''$ where $R'''$ is substituted or unsub-stituted alkyl, aryl, heteroaryl or alkenyl.

Preferably, $R^2$ in formula (I) is hydrogen; alkyl, such as methyl, ethyl or propyl; alkenyl, such as prop-2-enyl; alkynyl, such as prop-2-ynyl; halogenoalkyl, such as 2-fluoroethyl; hydroxyalkyl, such as 2-hydroxyethyl or 3-hydroxypropyl; cyanoalkyl, such as cyanomethyl; alkoxyalkyl, such as 2-methoxyethyl; or mercaptoalkyl, such as 2-mercaptoethyl. Most preferably, $R^2$ is alkynyl such as prop-2-ynyl.

(RiSys) in formula (I) may be a substituted or unsubstituted homocyclic or heterocyclic ring system having from 3 to 10 ring atoms, such as a phenyl, naphthyl or heterocyclyl ring system. When (RiSys) is heterocyclyl, (RiSys) is preferably a 5-membered or 6-membered aromatic heterocyclyl ring system containing from 1 to 2 heteroatoms selected from the group consisting of oxygen, nitrogen and sulfur, for example, thienyl, pyridyl, pyrimidinyl, thiazolyl or oxazolyl. Preferred heterocyclyl ring systems include thienyl or pyridyl. Most preferably, (RiSys) is substituted phenyl.

(RiSys) is preferably substituted with at least one electron withdrawing group, although (RiSys) may be substituted with at least one electron donating group such as a lower alkyl. For example, (RiSys) may be phenyl with a 3-methyl substituent.

Preferred substituents on (RiSys) include those selected from $R^3$ and $R^4$ where $R^3$ an $R^4$ are as follows:

$R^3$ may be hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms wherein the substituents may be, for example, halogeno, hydroxy, amino, alkoxy, alkylthio, alkylamino, dialkylamino or alkanoylamino substituents.

Preferably, $R^3$ is hydrogen; alkyl having from 1 to 6 carbon atoms such as methyl and ethyl; alkenyl having from 1 to 6 carbon atoms such as vinyl; alkynyl having from 1 to 6 carbon atoms such as ethynyl; halogeno such as fluoro, chloro, bromo and iodo; cyano; cyanato; thiocyanato; selenocyanato; azido; or

substituted alkyl having from 1 to 3 carbon atoms such as fluoromethyl (e.g. trifluoromethyl), hydroxymethyl, alpha-hydroxyethyl, aminomethyl, methoxymethyl, methylthiomethyl, methylaminomethyl, dimethylaminomethyl and acetylaminomethyl.

Most preferably, $R^3$ is hydrogen, alkyl having from 1 to 3 carbon atoms such as methyl and ethyl, ethynyl, trifluoromethyl, hydroxymethyl, alpha-hydroxyethyl, chloro, bromo or iodo.

$R^4$ may be hydrogen; substituted or unsubstituted alkyl, alkenyl, or alkynyl, each having from 1 to 6 carbons wherein the substituents may be, for example, halogeno, hydroxy, nitro, cyano, carboxyl, carbamoyl, alkoxycarbonyl, alkanoyl, alkenoyl, alkynoyl, aroyl, heterocyclyl, heterocycloyl, sufamoyl, alkylamino and trialkylammonio; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl alkylsulfinyl, alkylsulfonyl, alkenesulfinyl, alkenesulfonyl of up to 6 carbon atoms unsubstituted or bearing one or more substituents selected from halogeno, hydroxy, amino, alkylamino, dialkylamino, cyano, carboxyl, carbamoyl, alkoxycarbonyl, alkanoyl, alkenoyl, alkynoyl, aroyl, heterocyclyl, heterocycloyl, sulfamoyl; pentafluorothio; or nitro, sulfamoyl, (((carboxy)methyl)amino)sulfonyl, sulfinamoyl, halogeno, cyano, cyanato, thiocyanato, selenocyanato or azido; or dialkylphosphinoyl, diarylphosphinoyl, dialkoxyphosphoryl, diaryloxyphosphoryl, dialkylaminophosphoryl or diarylaminophosphoryl.

Typically, $R^4$ is hydrogen; substituted or unsubstituted alkyl having up to six carbon atoms such as methyl, ethyl, propyl, isopropyl, isobutyl, tert-butyl, pentyl, hexyl hydroxyalkyl (e.g., 2-hydroxyethyl and 3-hydroxypropyl), alkoxyalkyl (e.g., 2-methoxyethyl, 2-ethoxyethyl, 3-methoxypropyl and 2-methoxypropyl), mercaptoalkyl (e.g., 2-mercaptoethyl and 3-mercaptopropyl), alkylthioalkyl (e.g., 2-methylthioethyl, 3-methylthiopropyl and 2-ethylthioethyl), halogenoalkyl (e.g., 2-fluoroethyl, 2-chloroethyl, 2-bromoethyl, 3-fluoropropyl and 3-chloropropyl), cyanoalkyl (e.g., cyanomethyl, 2-cyanoethyl and 3-cyanopropyl), aminoalkyl (e.g., 2-aminoethyl, 3-aminopropyl and 3-amino-2-methylpropyl), alkylaminoalkyl (e.g., 2-methylaminoethyl, 2-ethylaminoethyl and 3-methylaminopropyl), dialkylaminoalkyl (e.g., 2-dimethylaminoethyl, 2-diethylaminoethyl and 3-dimethylaminopropyl), and alkanoylalkyl (e.g., acetonyl, 2-acetylethyl, propionylmethyl, 2-propionylethyl, 3-acetylpropyl and 4-acetylbutyl), carboxyalkyl (e.g., carboxymethyl and 2-carboxyethyl), carbamoylalkyl (e.g., carbamoylmethyl) or alkanoyl (e.g., acetyl, propionyl and butyryl); substituted or unsubstituted alkenyl having up to six carbons such as prop-2-enyl, but-2-enyl, but-3-enyl, 2-methylprop-2-enyl, hex-2-enyl, hex-5-enyl, 2,3-dimethylbut-2-enyl, hydroxyalkenyl, halogenoalkenyl, cyanoalkenyl, aminoalkenyl, alkylaminoalkenyl, alkanoylalkenyl, carboxyalkenyl and carbamoylalkenyl; substituted or unsubstituted alkynyl having up to six carbons such as prop-2-ynyl, but-2-ynyl, but-3-ynyl, pent-2-ynyl, 3-methylpent-4-ynyl, hex-2-ynyl, hex-5-ynyl, hydroxyalkynyl, halogenoalkynyl, cyanoalkynyl, aminoalkynyl, alkylaminoalkynyl, alkanoylalkynyl, carboxyalkynyl and carbamoylalkynyl; substituted or unsubstituted alkylsulfinyl; substituted or unsubstituted alkylsulfonyl having up to six carbon atoms such as methylsulfonyl and trifluoromethylsulfonyl; substituted or unsubstituted alkenesulfinyl having up to six carbon atoms such as butenesulfinyl; substituted or unsubstituted alkenesulfonyl having up to six carbon atoms such as pentenesulfonyl; substituted or unsubstituted aroyl such as benzoyl; substituted or unsubstituted heteroaroyl such as thenoyl; substituted or unsubstituted arylsulfinyl such as naphthylsulfinyl; substituted or unsubstituted arylsulfonyl such as phenylsulfonyl; heteroarylsulfinyl such as pyrazolylsulfinyl; heteroarylsulfonyl such as pyridylsulfonyl; pentafluorothio; nitro; sulfamoyl; (((carboxy)methyl)amino)-sulfonyls such as $-SO_2NHCH_2COOH$; sulfinamoyl; halogeno such as fluoro, chloro, bromo and iodo; cyano; cyanato; thiocyanato; selenocyanato; or azido.

Preferably, $R^4$ is hydrogen, fluoro, chloro, cyano, nitro, benzoyl, phenylsulfonyl, trifluoromethylsulfonyl, sulfamoyl, (((carboxy)methyl)amino)sulfonyl or pentafluorothio.

Preferred compounds are those where (RiSys) is a phenyl group having the structure

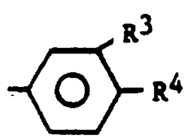

with $R^3$ and $R^4$ having the meaning indicated above.

Particularly preferred compounds of the invention capable of inhibiting thymidylate synthase may have the formula (III):

(III)

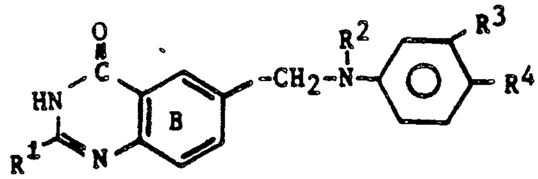

where:

$R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy;

$R^2$ is hydrogen, alkyl, alkenyl, alkynyl, halogenoalkyl, hydroxyalkyl, cyanoalkyl, alkoxyalkyl or mercaptoalkyl.

$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and

$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato or azido; or dialkylphosphinoyl, diarylphosphinoyl, dialkoxyphosphoryl, diaryloxyphosphoryl, dialkylaminophosphoryl or diarylaminophosphoryl.

Preferred such compounds include those where $R^1$ is alkyl or alkoxy, such as methyl or methoxy; $R^2$ is hydrogen, methyl, ethyl, propyl, prop-2-enyl, prop-2-ynyl, cyanomethyl, 2-fluoroethyl, 3-hydroxypropyl, 2-hydroxyethyl, 2-methoxyethyl or 2-mercaptoethyl; $R^3$ is hydrogen; alkyl or alkynyl, each having from 1 to 6 carbon atoms, such as methyl, ethyl or ethynyl; cyano; azido; halogeno, such as chloro, bromo or iodo; or substituted alkyl having from 1 to 3 carbon atoms, such as trifluoromethyl, hydroxymethyl or alpha-hydroxyethyl and $R^4$ is hydrogen; substituted or unsubstituted alkylsulfonyl, such as trifluoromethylsulfonyl; substituted or unsubstituted aroyl, such as benzoyl; substituted or unsubstituted arylsulfonyl, such as phenylsulfonyl; nitro; sulfamoyl; pentafluorothio; halogeno such as fluoro or chloro; cyano; or (((carboxy)-methyl)amino)sulfonyl.

Particularly preferred compounds capable of inhibiting thymidylate synthase have the formula (IV):

(IV)

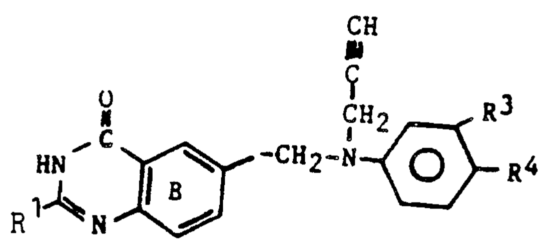

where:

$R^1$ is methyl, phenyl or methoxy;

$R^3$ is hydrogen, methyl, ethyl, ethynyl, trifluoromethyl, hydroxymethyl, alpha-hydroxyethyl, chloro, bromo, or iodo; and

$R^4$ is hydrogen, fluoro, chloro, cyano, nitro, benzoyl, phenylsulfonyl, trifluoromethylsulfonyl, sulfamoyl, ((-(carboxy)methyl)amino)sulfonyl or pentafluorothio.

Preferred compounds of formula (IV) are those where $R^1$ is methyl and:

$R^3$ is hydrogen and $R^4$ is chloro,
$R^3$ is methyl and $R^4$ is hydrogen,
$R^3$ is trifluoromethyl and $R^4$ is hydrogen,
$R^3$ is ethyl and $R^4$ is hydrogen,
$R^3$ is bromo and $R^4$ is hydrogen,
$R^3$ is hydroxymethyl and $R^4$ is hydrogen,
$R^3$ is alpha-hydroxyethyl and $R^4$ is hydrogen,
$R^3$ is hydrogen and $R^4$ is cyano,
$R^3$ is iodo and $R^4$ is hydrogen,
$R^3$ is chloro and $R^4$ is cyano,

$R^3$ is hydrogen and $R^4$ is nitro,
$R^3$ is ethynyl and $R^4$ is hydrogen,
$R^3$ is trifluoromethyl and $R^4$ is nitro,
$R^3$ is hydrogen and $R^4$ is benzoyl,
$R^3$ is trifluoromethyl and $R^4$ is cyano,
$R^3$ is hydrogen and $R^4$ is phenylsulfonyl,
$R^3$ is hydrogen and $R^4$ is trifluoromethylsulfonyl,
$R^3$ is hydrogen and $R^4$ is sulfamoyl,
$R^3$ is trifluoromethyl and $R^4$ is sulfamoyl,
$R^3$ is trifluoromethyl and $R^4$ is $-SO_2NHCH_2COOH$,
$R^3$ is trifluoromethyl and $R^4$ is phenylsulfonyl.

Also preferred are those compounds where $R^1$ is phenyl, $R^3$ is hydrogen and $R^4$ is chloro.

The invention also relates to a process for making a compound of the formula (I) as defined above comprising the steps of:

(1) allowing a compound of the formula $R_L$-$CH_2$-D to react with a compound of the formula

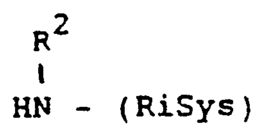

where D is a displaceable group, provided that one or more of $R_L$ $R^2$ and (RiSys) may contain a chemical group or groups which may be protected by a protecting group during the course of the reaction; and

(2) selectively removing one or more of any protecting groups in $R_L$, $R^2$ and (RiSys).

The process of the invention allows production of compounds of formula (I) capable of inhibiting thymidylate synthase. The compounds which may be produced by this process include all those formula (I) compounds described above.

The groups $R_L$, $R^2$ and (RiSys) employed in the starting materials include all those $R_L$, $R^2$ and (RiSys) groups described above.

Displaceable group D may be halogeno, such as fluoro, chloro or bromo, or substituted sulfonyloxy, such as methanesulfonyloxy, trifluoromethanesulfonyloxy, toluene-p-sulfonyloxy or 4-bromobenzenesulfonyloxy. Preferably, D is bromo.

A preferred process for the preparation of a quinazoline of the invention is by the reaction of a compound of the formula:

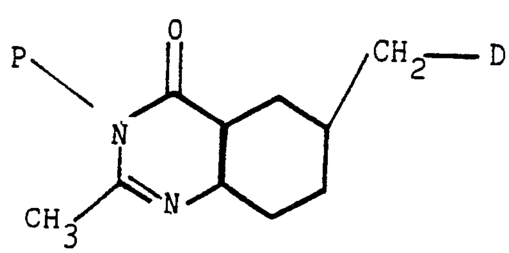

wherein P is hydrogen or a protecting group and D is a displaceable group with a compound of the formula:

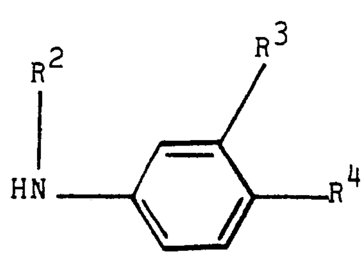

wherein $R^2$, $R^3$, and $R^4$ are as defined above. It should be noted that, when $R^2$, $R^3$ and $R^4$ contain a chemical group or groups which need(s) protecting in the course of the synthesis, then such group(s) may

be protected by (a) conventional protecting group(s). After completion of the reaction, any undesired protecting group(s) in $R^2$, $R^3$, and $R^4$ is (are) removed.

A suitable moiety for P above when it is a protecting group is, for example, a pivaloyloxymethyl group, which may be removed by hydrolysis with a base such as sodium hydroxide; a tert-butyloxycarbonyl group, which may be removed by hydrolysis with an acid such as hydrochloric acid or with a base such as lithium hydroxide; or a 2-(trimethylsilyl)ethoxymethyl group, which may be removed by a fluoride salt such as tetra-n-butyl ammonium fluoride or with an acid such as hydrochloric acid.

A suitable protecting group for a hydroxyl group is, for example, an esterifying group such as an acetyl or benzoyl group which may be removed by hydrolysis with a base such as sodium hydroxide. Alternatively, when other groufps present in the starting material do not contain an alkenyl or alkynyl group, the protecting group may be, for example, an alpha-arylalkyl group, such as a benzyl group, which may be removed by hydrogenation in the presence of a catalyst such as palladium-on-charcoal.

A suitable protecting group for a mercapto group is, for example, an esterifying group such as an acetyl group, which may be removed by hydrolysis with a base such as sodium hydroxide.

A suitable protecting group for an amino group may be, for example, an alkoxycarbonyl group, such as a tert-butyloxycarbonyl group, which may be removed by treatment with an organic acid such as trifluoroacetic acid. Alternatively, the protecting group may be a benzyloxycarbonyl group, which may be removed by treatment with a Lewis acid such as boron tris(trifluoroacetate).

A suitable alternative protecting group for a primary amino group is, for example, a phthaloyl group which may be removed by treatment with an alkylamine such as dimethylaminopropylamine or with hydrazine.

A suitable protecting group for a carboxy group may be an esterifying group, for example, a methyl or an ethyl group, which may be removed by hydrolysis with a base such as sodium hydroxide. Another useful protecting group is a tert-butyl group, which may be removed by treatment with an organic acid such as trifluoroacetic acid.

Preferred protecting groups include an esterifying group, an alpha-arylalkyl group, a substituted or unsubstituted alkoxycarbonyl group, a phthaloyl group, a pivaloyloxymethyl group or a methyl oxyether-type group, such as methoxymethyl or 2-(trimethylsilyl)ethoxymethyl.

The compound of the formula

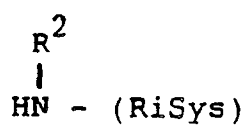

may be prepared by the reaction of the bromide, chloride or tosylate of $R^2$ with a compound of formula (V),

(V) $H_2N$ -·(RiSys)

Use of the tosylate of $R^2$, especially when $R^2$ is prop-2-ynyl, in the reaction with a compound of formula (V) unexpectedly avoids alkylation of any unprotected hydroxyl groups present on (RiSys).

The compound

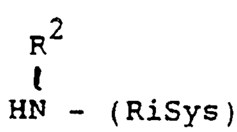

may be treated with a base, e.g., sodium hydride, prior to being brought into contact with the compound $R_L$-$CH_2$-D. Alternatively, the compound

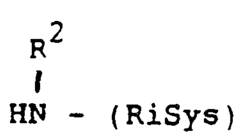

may be prepared by treating the compound $CF_3CONH$-(RiSys) with a base and contacting the resulting first intermediate compound with $R^2$-D where D is a displaceable group as previously described to form a second intermediate compound, isolating the second intermediate compound and hydrolyzing the second

intermediate compound. Exemplary bases include potassium carbonate, calcium carbonate, potassium hydride, lithium hydride, potassium tertiary butoxide, sodium disilazane, lithium disilazane, potassium disilazane, and lithium diisopropylamide.

Further, the compound

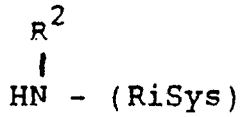

may also be prepared by treating D-(RiSys) with $R^2NH_2$, where D is a displaceable group as previously described and where the chemical nature of (RiSys) activates the group D. By "activation" of the group D is meant activation for the displacement of D. For example, if (RiSys) is substituted with a strong electron withdrawing group, the displacement of D is typically facilitated.

Another aspect of the invention relates to a pharmaceutical composition comprising a pharmaceutically acceptable diluent or carrier in combination with an amount effective to inhibit thymidylate synthase of a compound of formula (I), as described above. The composition preferably contains an amount of the formula (I) compound which is a nontoxic amount.

The formula (I) compounds of the invention which may be employed in the composition of the invention include all those formula (I) compounds described above, as well as the pharmaceutically acceptable salts of these compounds. Pharmaceutically acceptable acid addition salts of the compounds of the invention containing a basic group are formed where appropriate with strong or moderately strong organic or inorganic acids in the presence of a basic amine by methods known to the art. Exemplary of the acid addition salts which are included in this invention are maleate, fumarate, lactate, oxalate, methanesulfonate, ethanesulfonate, benzenesulfonate, tartrate, citrate, hydrochloride, hydrobromide, sulfate, phosphate and nitrate salts. Pharmaceutically acceptable base addition salts of compounds of the invention containing an acidic group are prepared by known methods from organic and inorganic bases and include nontoxic alkali metal and alkaline earth bases, for example, calcium, sodium, and potassium hydroxide; ammonium hydroxide, and nontoxic organic bases such as triethylamine, butylamine, piperazine, and tri-(hydroxymethyl)methylamine.

As stated above, the compounds of the invention possess antiproliferative activity, a property which may express itself in the form of antitumor activity. A compound of the invention may be active per se or it may be a pro-drug that is converted in vivo to an active compound. Preferred compounds of the invention, such as quinazolines, are active in inhibiting the growth of the L1210 cell line, a mouse leukemia cell line which can be grown in tissue culture. Such compounds of the invention are also active in inhibiting the growth of bacteria such as E.Coli, a gram-negative bacterium which can be grown in culture.

The compounds of formula (I) and pharmaceutically acceptable salts thereof may be incorporated into convenient dosage forms such as capsules, tablets, or injectable preparations. Solid or liquid pharmaceutically acceptable carriers may be employed. Solid carriers include starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. When a liquid carrier is used, the preparation may be in the form of a syrup, elixir, emulsion, soft gelatin capsule, sterile injectable liquid (e.g. solution), such as an ampoule, or an aqueous or nonaqueous liquid suspension.

The pharmaceutical preparations are made following conventional techniques of a pharmaceutical chemist involving steps such as mixing, granulating, and compressing, when necessary, for tablet forms, or mixing, filling and dissolving the ingredients, as appropriate, to give the desired oral or parenteral products.

The compositions of the invention may further comprise one or more other compounds which are antitumor agents, such as mitotic inhibitors (e.g., vinblastine), alkylating agents (e.g., cis-platin, carboplatin and cyclophosphamide), DHFR inhibitors (e.g., methotrexate, piritrexim or trimetrexate), antimetabolites (e.g., 5-fluorouracil and cytosine arabinoside), intercalating antibiotics (e.g., adriamycin and bleomycin), enzymes (e.g., asparaginase), topoisomerase inhibitors (e.g., etoposide) or biological response modifiers (e.g., interferon).

The composition of the invention may also comprise one or more other compounds including antibacterial, antifungal antiparasitic, antiviral, antipsoriatic and anticoccidial agents. Exemplary antibacterial agents include, for example, sulfonamides such as sulfamethoxazole, sulfadiazine, sulfameter or sulfadoxine; DHFR inhibitors such as trimethoprim, bromodiaprim, or trimetrexate; pencillins; cephalosporins; aminoglycosides; bacteriostatic inhibitors of protein synthesis; the quinolonecarboxylic acids and their fused

isothiazolo analogs.

Another aspect of the invention relates to a therapeutic process of inhibiting thymidylate synthase which process comprises administering to a vertebrate host, such as a mammal or bird, an amount effective to inhibit thymidylate synthase of a compound of the formula (I) described above. The compounds of the invention are particularly useful in the treatment of mammalian hosts, such as human hosts, and in the treatment of avian hosts.

Any of the formula (I) compounds described above, or pharmaceutically acceptable salts thereof, may be employed in the therapeutic process of the invention. The compounds of the invention may be administered in the therapeutic process of the invention in the form of a pharmaceutically acceptable composition comprising a diluent or carrier, such as those described above. Doses of the present compounds of formula (I), or pharmaceutically acceptable salts thereof, preferably include pharmaceutical dosage units comprising an efficacious, nontoxic quantity of active compound. By an efficacious quantity is meant a quantity sufficient to inhibit TS and derive the beneficial effects therefrom through administration of one or more of the pharmaceutical dosage units. An exemplary daily dosage unit for a vertebrate comprises an amount of up to about 5,000 mg of active compound per square meter of the body area of the vertebrate host.

The selected dose may be administered to a warm-blooded animal or mammal, for example a human patient, in need of treatment mediated by thymidylate synthase inhibition by any known method of administration, including topically (e.g. as an ointment or cream), orally, rectally (e.g. as a suppository), parentally, by injection, or continuously by infusion, intravaginally, intranasally, intrabronchially, intra-aurally or intraocularly.

The compounds of formula (I) may be further characterized as producing any one or more of an antiproliferative effect, an antibacterial effect, an antiparasitic effect, an antiviral effect, an antipsoriatic effect, an antiprotozoal effect, an anticoccidial effect or an antifungal effect. The compounds of formula (I) are especially useful in producing an antitumor effect in a vertebrate host harboring a tumor.

The compounds of formula (I) are antagonists of a folate cofactor and therefore may affect one or more other folate-dependent enzymatic systems as well. Examples of other folate-dependant enzymatic systems which may be affected include 5,10-methylenetetrahydrofolate reductase, serine hydroxymethyltransferase, and glycineamineribotide transformylase.

When the compounds of the invention comprise an $R_L$ group such as the quinazoline rings disclosed in European Patent Application No. 239,362, which is incorporated herein by reference, that is, quinazoline rings of the structure

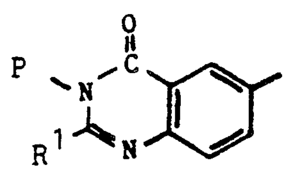

where $R^1$ is alkyl, cycloalkyl, alkenyl, alkynyl, alkoxy, alkythio, aryl, aryloxy, arylalkyl, halogeno, hydroxy, amino, mercapto, pyridylthio, pyrimidinylthio, or substituted alkyl or alkoxy and P is hydrogen or a protecting group, then (RiSys) need not be (i) -Ar-CONHR$^E$ where $R^E$ is such that $R^E$-$NH_2$ is an amino acid and where Ar is substituted or unsubstituted phenyl, naphthyl or heterocyclyl, or (ii) -Ar-$CO_2$H where Ar is as previously described or a reactive derivative of the acid such as an acyl halide, a mixed anhydride, an acyl azide or the product of the reaction of the acid and a carbodiimide, or a pharmaceutically acceptable salt or ester thereof.

The following examples illustrate the invention, although the scope and spirit of the invention is not limited thereto.

## EXAMPLES

The structures of all compounds of the invention were confirmed by proton magnetic resonance spectroscopy, infrared spectroscopy, elemental microanalysis and, in certain cases, by mass spectrometry.

Proton magnetic resonance spectra were determined using a General Electric QE-300 spectrometer operating at a field strength of 300 MHz. Chemical shifts are reported in parts per million (delta) downfield

from tetramethysilane as an internal standard and peak multiplicities are designated as follows: s, singlet; d, doublet; dd, doublet of doublets; t, triplet; br s, broad singlet; br d, broad doublet, br, broad signal; m, multiplet.

Mass spectra were determined using a VG 7070E-HF high resolution mass spectrometer using the direct insertion method, an ionizing voltage of 70 eV, and an ion source temperature of 200 °C. Infrared absorption spectra were taken on a Perkin-Elmer 457 spectrometer. Elemental microanalysis gave results for the elements stated within ± 0.4% of the theoretical values.

General procedures were as follows:

Propargyl bromide was used as an 80% w/w solution in toluene.

N,N-Dimethylformamide ("DMF") was dried over activated (250 °C) 3-Å molecular sieves; N,N-dimethylacetamide ("DMA") (Aldrich Gold Label grade) was similarly dried. Tetrahydrofuran ("THF") was distilled from sodium benzophenone ketyl under nitrogen. Ether refers to diethyl ether. Petrol refers to petroleum ether of bp 36-53 °C.

Flash chromatography was performed using Silica gel 60 (Merck Art 9385). Where the crude solid (x g) was insoluble in the chosen eluant, it was dissolved in a more polar solvent and Merck Art 7734 silica (4x g) added. The slurry was evaporated to dryness on a rotary evaporator fitted with a coarse glass frit to prevent spraying of the silica. The coated silica was then applied to the column. Thin layer chromatographs ("TLC") were performed on precoated sheets of silica $60F_{254}$ (Merck Art 5719). Extracts were dried over $Na_2SO_4$ or $MgSO_4$. Melting points were determined on a Mel-Temp apparatus and are corrected.

All of the starting anilines used were commercially available with the following five exceptions:

(i) 4-Aminodiphenylsulfone was prepared from 4-nitrodiphenylsulfone as described by W.R. Waldron and E.E. Reid (Journal of the American Chemical Society, 45, 2399, (1923)).

(ii) 4-Cyano-3-(trifluoromethyl)aniline was prepared by the method of L. Friedman and H. Shechter (J. Org. Chem. 26, 2522, 1961)): A mixture of 5-amino-2-bromobenzotrifluoride (12.00 g, 50 mmol), CuCN (5.37 g, 60 mmol), and DMF (7.5 mL) was heated under reflux for 2 h. It was poured into 30% w/v NaCNaq (150 mL) and extracted with ether (75 mL). The ether layer was washed sequentially with 10% NaCNaq (100 mL), $H_2O$ (100 mL), and brine (100 mL), dried ($Na_2SO_4$) and evaporated to dryness to give the crude product (8.00 g). This was flash chromatographed on silica (700 g) using 20% ether in $CH_2Cl_2$ as the eluant to give the pure product as a light tan solid (7.03 g, 76%) suitable for further use. An analytical sample recrystallized from $EtOH/H_2O$ as off-white needles, mp 142-143 °C, NMR satisfactory, Anal. ($C_8H_5F_3N_2$) C,H,N,F.

(iii) 4-Amino-2-trifluoromethyldiphenylsulfone.

A mixture of sodium benzenesulfinate (18.06 g, 0.11 mol), ethylene glycol (75 ml) and 2-(2-ethoxyethoxy)ethanol (120 ml) was heated to 125 °C in a flask equipped with a overhead stirrer. 2-Fluoro-5-nitrobenzotrifluoride (20.91 g, 0.1 mol) was added and the mixture was heated at 135 °C for 3.5 h. The resulting solution was cooled and $H_2O$ (20 mL) was added to throw down an oil which eventually solidified. The pasty solid was filtered off, washed with hot $H_2O$ (75 mL), partially dried, and recrystallized from EtOH-$H_2O$ with carbon treatment to provide a tan-colored solid (7.85 g, 23.7%). Flash chromatography (70% $CH_2Cl_2$ in petrol) of a small portion gave the analytical sample of 4-nitro-2-trifluoromethyldiphenylsulfone as a cream-colored solid mp 167-168 °C, NMR satisfactory, Anal. ($C_{13}H_8F_3NO_4S$) C,H,N,F,S.

4-Nitro-2-trifluoromethyl-diphenylsulfone (7.00 g, 21 mmol) was added during 4 min. to a warm solution of $SnCl_2$-$2H_2O$ (28.43 g, 126 mmol) in EtOH (33 mL). Heating for 8 min. at 54 °C gave a solution to which conc. HC1 (33 mL) was added. The ethanol was boiled away during 20 min., the residue was cooled in ice and treated with 2.5 N NaOH to pH 14 to precipitate the product which was filtered off, washed with water, and dried (5.62 g, 89%) - suitable for further use. The analytical sample was obtained by flash chromatography (3% $CH_3CN$ in $CH_2Cl_2$) as a white solid mp 186-188 °C, NMR satisfactory, Anal. ($C_{13}H_{10}F_3NO_2S$) C,H,N,F,S.

(iv) 4-Amino-2-(trifluoromethyl)benzenesulfonamide

2-Amino-5-nitrobenzotrifluoride (82.45 g, 0.4 mol) was added to a mechanically stirred, almost boiling mixture of conc. HCl (120 mL) and $H_2O$ (120 mL) in a 2 L beaker. Glacial HOAc (200 mL) was added a give a clear yellow solution which was then cooled to -10 °C; the yellow hydrochloride salt precipitated. A solution of $NaNO_2$ (29.67 g, 0.43 mol) in $H_2O$ (200 mL) was added during 15 min. keeping the temperature

below -5° C, and the resulting mixture was stirred at -5 to -10° C for 15 min. Meanwhile, glacial HOAc (400 mL) contained in a 4 L beaker was saturated (45 min.) with $SO_2$ gas and CuCl (9.90 g, 0.1 mol) then added. Passage of $SO_2$ gas was continued for 30 min. until most of the solid had dissolved to give a thin blue-green suspension which was then cooled to 5° C. The diazonium preparation was added to the cuprous preparation during 11 min., causing vigorous evolution of nitrogen; the resulting green mixture was stirred for 1.5 h while it attained ambient temperature. Crushed ice (1 Kg) was added to give the product as a flocculent yellow solid, 4-nitro-2-(trifluoromethyl)benzenesulfonyl chloride which was filtered off and washed well with water and dried in vacuo (51.36 g). Careful recrystallization from cyclohexane (400 mL) with seeding gave the pure product as cubic crystals (38.26 g, 33%) mp 81-83° C. NMR ($CDCl_3$) satisfactory. Anal. ($C_7H_3ClF_3NO_4S$) C,H,N,Cl.

4-Nitro-2-(trifluoromethyl)benzenesulfonyl chloride (11.58 g, 40 mmol) dissolved in EtOH (150 mL) was treated with conc. $NH_4OH_{aq}$ (13.5 mL, 200 mmol). The mixture was kept for 1 h. at ambient temperature, quenched with $H_2O$ (600 mL) and acidified to pH 4 with 1N HCl to give a precipitate which was kept thus overnight at 4° C. The product 4-nitro-2-(trifluoromethyl)benzenesulfonamide was filtered off and washed and dried to give pale yellow needles (7.54 g, 69.8%) mp 163-164° C, NMR ($Me_2SO$-$d_6$) satisfactory. Anal. ($C_7H_5N_2F_3O_4S$) C,H,N,F,S.

4-Nitro-2-(trifluoromethyl)benzenesulfonamide (6.89 g, 25.5 mmol) was added during 4 min. to a warm solution of $SnCl_2$-$2H_2O$ (28.77 g, 127.5 mmol) in EtOH (35 mL). The mixture was heated to 55° C for 10 min. to give a solution to which conc. HCl (35 mL) was added. The ethanol was boiled away during 20 min., and the mixture was cooled and treated with 2.5 N NaOH to pH 6.5 and then extracted with EtOAc (3 x 250 mL). The combined extracts were washed with brine, dried, and evaporated to dryness to give the product, 4-nitro-2-(trifluoromethyl)benzenesulfonyl chloride (5.44 g, 89%). An analytical sample was recrystallized from $H_2O$ to give beige plates, mp 186-187° C, NMR ($Me_2SO$-$d_6$) satisfactory, Anal. ($C_7H_7F_3N_2O_2S$) C,H,N,S.

(v) Methyl N-(4-amino-2-(trifluoromethyl)benzenesulfonyl)glycinate.

4-Nitro-2-(trifluoromethyl)benzenesulfonyl chloride (13.03 g, 45 mmol) dissolved in EtOH (175 mL) was added during 3 min. to a slurry formed from methyl glycinate hydrochloride (11.30 g, 90 mmol) in a mixture of EtOH (30 mL) and $Et_3N$ (25.1 ml, 180 mmol) keeping the temperature between 0 and 5° C. The mixture was stirred for 50 min. at 20° C and then poured into $H_2O$ (1600 mL) to precipitate the product, methyl N-(4-nitro-2-(trifluoromethyl)benzenesulfonyl)glycinate. The pH was adjusted to 1.5 with 1N HCl and the white needles were filtered off, washed with water, and dried (9.28 g, 60%) mp 139.5-140° C. NMR ($Me_2SO$-$d_6$) satisfactory. Anal. ($C_{10}H_9F_3N_2O_6S$) C,H,N,F,S.

Methyl N-(4-Nitro-2-(trifluoromethyl)benzenesulfonyl)glycinate (7.87 g, 23 mmol), was added during 4 min. to a warm solution of $SnCl_2$-$2H_2O$ (25.95 g. 115 mmol) in MeOH (35 mL). The mixture was heated to 55° C for 10 min. to give a solution to which conc. HCl (35 mL) was added. The ethanol was boiled away during 20 min., and the mixture was cooled and treated with 2.5 N NaOH. Basification to pH 6.0 was carefully done. The mixture was then extracted with EtOAC (3 X 250 mL). The combined extracts were washed with brine, dried, and evaporated to dryness to give the product. The product, methyl N-(4-amino-2-(trifluoromethyl)benzenesulfonyl)glycinate, was obtained as a white solid (3.46 g, 48%). An analytical sample was recrystallized from $H_2O$ to give beige crystals, mp 133-134° C, NMR ($Me_2SO$-$d_6$) satisfactory, Anal. ($C_{10}H_{11}F_3N_2O_4S$) C,H,N,F,S.

The propargylaniline intermediates listed in Table I below were prepared by the following general method:

The starting aniline and propargyl bromide were allowed to react in the solvent described in Table I in the presence of potassium carbonate. Quantities and reaction conditions are also shown in Table I. The reaction was stopped when TLC assay showed optimal formation of the desired secondary amine; the corresponding dipropargylaniline was typically present as a by-product along with some unreacted starting aniline. 4-(Dimethylamino)cinnamaldehyde was useful as a spray reagent in differentiating these amines. The reaction mixture was poured into water and the organic material extracted with ether. The washed and dried extract was evaporated in vacuo to provide the crude product. This was flash chromatographed (using a weight of silica about 50 to 100 times that of the crude product) with the eluant as defined in Table I. In each case, the pure propargylamine thus obtained had a satisfactory NMR spectrum and a satisfactory elemental microanalysis for the elements stated.

The preparation of various propargyl anilines not appearing in Table I are provided below.

(i) N-(Prop-2-ynyl)-3-(hydroxymethylaniline)

A mixture of 3-aminobenzyl alcohol (3.42 g, 24.76 mmol), propargyl tosylate (L. Brandsma and H.D. Verkruijsse, "Synthesis of Acetylenes, Allenes and Cumulenes", Elsevier, Amsterdam, 1981, p. 223) (5.21 g, 24.76 mmol), potassium carbonate (3.42 g, 24.76 mmol), and DMF (75 mL) was stirred at ambient temperature for 24 h. The reaction mixture was partitioned between ether (250 mL) and $H_2O$ (250 mL). The organic layer was washed with water (2 x 250 mL) and then extracted with 0.5N HCl (200 mL). The acidic extract was basified with 5N NaOH and extracted with ether (2 x 200 mL). The combined ether layers were dried ($Na_2SO_4$) and evaporated to yield the crude solid product (1.11 g, 28%). This, combined with a previously prepared sample (0.59 g) was purified by flash chromatography (5% MeOH in $CHCl_3$) to yield the pure product as a solid (1.27 g) mp 77-79° C, NMR satisfactory. Anal. ($C_{10}H_{11}NO$) C,H,N.

(ii) N-(Prop-2-ynyl)-3-(1-hydroxyethyl)aniline.

The above reaction was repeated with 3-(1-hydroxyethyl)aniline (4.00 g, 29.15 mmol), propargyl tosylate (6.13 g, 29.15 mmol), $K_2CO_3$ (4.03 g, 29.15 mmol) and DMF (75 mL). The product as first isolated from the ether extract was pure, and chromatography was unnecessary. A thick oil was obtained which crystallized when stored at 0° C (1.83 g, 36%), NMR satisfatory. Anal. ($C_{11}H_{13}NO$) C,H,N.

(iii) N-(Prop-2-ynyl)-4-chloroaniline

4-chlorotrifluoroacetanilide (Bourne, E.J.; Henry, S.H.; Tatlow, C.E.M.; Tatlow, J.C.; J. Chem. Soc. 1952, 4014.) (111.79 g, 0.50 mole) dissolved in DMF (100 mL) was added to sodium hydride (15.00 g, 0.625 mole) slurried in DMF (200 mL) during 30 min., keeping the temperature at 10-15° C. The mixture was stirred at 10° C for 45 min. Propargyl bromide (74.36 g, 0.50 mole) was then added during 10 min. at 8° C. The mixture was stirred for 2 hours as it attained ambient.temperature and then left to stand thus overnight for 19.5 h. The mixture was partitioned between ether (2L) and $H_2O$ (2L). The ether layer was washed with $H_2O$ (3 x 2 L) and evaporated to give a brown oil (139.7 g). This oil was treated with a solution of KOH (84.16 g, 1.50 mole) in $CH_3OH$ (750 ml) and the whole then heated under reflux for 3 h. Most (500 ml) of the $CH_3OH$ was evaporated in vacuo leaving a residue which was partitioned between ether (1.5 L) and $H_2O$ (1.5 L). The ether extract was washed with satd. saturated brine (2 x 1 L), dried ($MgSO_4$), and evaporated to dryness in vacuo. The resulting oil (79.3 g) was vacuum distilled to give a fore-run of about 20 g followed by the product (bp 126° C/5 mm) as a colorless oil (46.9 g, 56.6%). Anal. ($C_9H_8ClN$) C,H,N,Cl.

(iv) 4-Nitro-N-(prop-2-ynyl)aniline

This aniline was prepared from 1-fluoro-4-nitrobenzene by a published method (A.F. Garito, C.J. Horner, P.S. Kalyanaraman, and K.N. Desai, Makromol. Chemie, 181, 1605, (1980).) conducted on a twenty times scale (0.2 mole) and with a modified work-up: The reaction mixture was filtered, the solids washed with DMSO (100 mL) and the combined filtrates poured into chilled water to precipitate the crude product. This was isolated (29.16 g) and recrystallized from xylene (about 270 mL) to provide a first crop (9.9 g) and by partial evaporation of the mother liquor, a second crop (1.7 g). These crops were slurried in warm xylene, cooled, and collected to give a product (10.1 g, 29%), mp 139-144° C suitable for further use.

(v) 4-(Trifluoromethylsulfonyl)-N-propargylaniline

Trifluoromethanesulfonyl anhydride (50 g, 177 mmol) was syringed during 10 min. into a mixture of fluorobenzene (49.84 mL, 531 mmol) and aluminum chlorine (29.37 g, 220 mmol), and stirred at 0° C under argon. The resulting orange-colored mixture was stirred for 10 min. at 0° C and then for 23 h. at 25° C. The mixture was partitioned between $H_2O$ (1000 mL) and ether (2 x 400 mL). The combined ether layers were washed with $H_2O$ (2 x 500 mL) and brine (800 mL) and dried ($Na_2SO_4$) to give the crude product as an orange-colored oil (17.5 g). This was chromatographed on $SiO_2$ (1 Kg) using ether/$CCl_4$ (1.5/98.5) as the eluant. Fractions containing the second component to elute were pooled and evaporated to give the technical quality product (3.69 g, 9.1%). This was 88% pure by NMR ($CDCl_3$)delta: 7.37 (dd, 2H, $J_{ortho}$ =

8.9 & $J_{fluorine}$ = 8.1 Hz, $H^2$ & $H^6$), 8.09 (dd, 2H, $J_{ortho}$ = 8.9 & $J_{fluorine}$ = 4.9 Hz, $H^3$ & $H^5$). The single impurity was 12% of 3-(trifluoromethylsulfonyl)fluorobenzene delta:
7.55 (dddd, 1H, $J_{6,F}$ = 10.7, $J_{6,5}$ = 7.7, $J_{6,4}$ = 2.5 & $J_{6,2}$ = 1.0 Hz, $H^6$),
7.70 (ddd, 1H, $J_{5,4}$ = $J_{5,6}$ = 7.7 & $J_{5,F}$ = 5.0 Hz, $H^5$),
7.76 (ddd, 1H, $J_{4,5}$ = 7.7, $J_{4,6}$ = 2.5&$J_{4,2}$ = 2.2 Hz, $H^4$),
7.87 (br d, 1H, $J_{2,F}$ = 7.8 Hz, $H^2$).

A solution of 4-(trifluoromethanesulfonyl)fluorobenzene (technical quality, 88%, 1.16 g, 4.47 mmol) and propargylamine (0.51 g, 9.26 mmol) in DMSO (15 mL) was stirred over $K_2CO_3$ (0.80 g, 5.79 mmol) at 25° C for 24 h. The mixture was poured into $H_2O$ (400 mL), basified with 0.1 N NaOH to pH 11, and extracted with ether (2 x 150 ml). The combined organic layers were washed with brine (400 mL), dried ($Na_2SO_4$) and evaporated to give an essentially pure amber oil which slowly crystallized (0.79 g, 67%). A small sample was flash chromatographed on $SiO_2$ using $CH_2Cl_2$ as the eluant to give the analytical sample as a white solid, mp 86-87° C.

NMR ($CDCl_3$) delta:
2.31 (t, 1H,J=2.4 Hz, :::CH), 4.04 (d, 2H, J = 2.4 Hz, $CH_2$-C:::), 4.4-5.2 (br, 1H, NH), 6.77 (d, 2H, J = 8.9 Hz, $H^2$ & $H^6$), 7.82 (d, 2H, J = 8.9 Hz, $H^3$ & $H^5$). Anal. ($C_{10}H_8F_3NO_2S$) C,H,N,F,S. (Note: "C:::" denotes a carbon bonded to an adjacent atom, such as carbon or nitrogen, by a triple bond.)

(vi) 4-(Phenylsulfonyl)-N-(prop-2-ynyl)aniline.

A solution of 4-(phenylsulfonyl)fluorobenzene (40.00 g, 0.169 mol) and propargylamine (79.6 g, 1.445 mol, 8.55 equiv) in $Me_2SO$ (900 mL) containing $CaCO_3$ (18.6 g, 0.186 mol) in suspension was heated at 125° C for 32 h. with magnetic stirring and under an argon atmosphere. The mixture was cooled and filtered through celite. The filtrate was evaporated to dryness in vacuo to leave a residue which was partitioned between $H_2O$ (2 L) and $CH_2Cl_2$ (2 L). The organic layer was dried and evaporated to dryness to give the crude product (89.6 g). This was thrice flash chromatographed with $CH_3CN$ on silica (3 x 1 Kg) taking pure product out in the first two stages and re-chromatographing the fractions containing less pure material. The total yield was 34.53 g (75.3%) of a pale yellow solid identical by NMR to the product (Table I) prepared by a different route.

Examples of processes for preparing the compounds of the invention are described in Table II. For example, a number of quinazolines were prepared by the reaction of the appropriate propargylaniline with 6-bromomethyl-3,4-dihydro-2-methyl-4-oxoquinazoline (European Patent Application No. 87302525.8, published September 30, 1987) using the following general method: A solution of the proparglyaniline and the bromomethylquinazoline in the solvent stated was stirred over $CaCO_3$. The temperature and the duration of the reaction were as stated in Table II. The reaction was followed by TLC ($SiO_2$GF/EtOAc was in most cases a useful system) and the Epstein spray reagent (Epstein, J.; Rosenthal, R.W.; Ess R.J. Anal. Chem. 1955, 27 1435) was used to monitor consumption of the bromomethylquinazoline. The mixture was filtered under vacuum through a bed of celite to remove solid material and the filtrate was poured into iced water (1 L). The product precipitated and, in most cases, was filtered off on a Whatmans Number 5, washed well with $H_2O$, sucked dry, and then dried over $P_2O_5$ in vacuo to constant weight. In three cases, filtration was unsuccessful and the product was therefore extracted with a solvent. The extract was then washed with water and with brine, dried, and evaporated to dryness to give the crude product. Eight of the compounds were purified by recrystallizations, ten by flash chromatography, and two by reprecipitation. In some of the recrystallizations; $MgSO_4$ in equal weight to the crude product was used to dry the hot solution (and removed at the filtration stage); this art gave improved crystal formation.

All of the compounds in Table II gave satisfactory $^1H$ and $C^{13}$ NMR spectra. The $^1H$ spectrum of N-(-(3,4-dihydro-2-methyl-4-oxo-6-quinazolinyl)methyl-N-(prop-2-ynyl)-4-chloroaniline (the second compound in Table II) is provided to serve as an example, as follows: (solvent $Me_2SO-d_6$)delta:
2.33 (s, 3H, $CH_3$),
3.22 (t, 1H, J = 2.0 Hz, :::CH),
4.24 (d, 2H, J = 2.0 Hz, $CH_2$-C:::),
4.66 (s, 2H, $CH_2$),
6.81 (d 2H, J = 9.1 Hz, aromatic),
7.21 (d, 2H, J = 9.1 Hz, aromatic),
7.54 (d, 1H, J = 8.3 Hz, $H^8$),
7.69 (dd, 1H, J = 8.3 & 1.7 Hz, $H^7$),
7.95 (d, 1H, J = 1.7 Hz, $H^5$),

12.20 (br s, 1H, NH).

In like vein, the mass spectrum of this compound typifies the series: m/z 339 and 337 ($M^+$); 173 (M-$C_3H_7C1N$); 132 (173-$CH_3C$:::N).

Compounds prepared by methods other than by the general procedure described above are given below.

(i) N-((3,4-Dihydro-2-methyl-4-oxo-6-quinazolinyl)methyl)-N-(prop-2-ynyl)-4-trifluoromethanesulfonylaniline. - (Compound A in Tables III and IV below.)

Sodium hydride (60% oil dispersion, 0.71 g, 1.77 mmol) was placed in a flame dried flask and covered with argon. 4-(Trifluoromethylsulfonyl)-N-propargylaniline (0.453 g, 1.72 mmol) in DMF (9 mL) was cannulated in during 1 min. with stirring. An orange-red solution formed which was stirred for 30 min. To it was then added 6-bromomethyl-3,4-dihydro-2-methyl-4-oxo-3-((pivaloyl)oxy)methylquinazoline (European Patent Application No. 87302525.8, published September 30, 1987) (0.316 g, 0.86 mmol) in DMF (10 mL) and the solution was stirred at 25°C for 22.5 h. The mixture was poured into saturated $NaHCO_3$ aq. (100 mL) and extracted with $CH_2Cl_2$ (4 x 100 mL). The combined organic layers were dried ($Na_2SO_4$) and evaporated to dryness under oil pump vacuum. The brown residue was treated with MeOH (20 mL) followed by 1N LiOH (2 mL, 2 mmol) and the resulting mixture was stirred for 37 min., acidified to pH3 with 1N HCl, poured into $H_2O$ (100 mL), and extracted with $CH_2Cl_2$ (4 x 50 mL). The combined, dried extract was taken to dryness to give the crude product (0.350 g) which was loaded in 1,2-dichloroethane onto a silica column (60 g) and eluted off with 40% $CH_3CN$ in $CCl_4$. Evaporation of the appropriate fractions gave the pure product as a white solid (0.127 g, 34%) mp 250-251.5°C, NMR ($Me_2SO$-$d_6$) delta:
2.33 (s, 3H, $CH_3$),
3.34 (t, 1H, J = 2.2 Hz, :::CH),
4.50 (d, 2H, J = 2.2 Hz, $CH_2$-C:::),
4.93 (s, 2H, $CH_2$),
7.07 (d, 2H, J = 9.2 Hz, aromatic)
7.56 (d, 1H, J = 8.4 Hz, $H^8$),
7.68 (dd, 1H, J = 8.4 & 2.0 Hz, $H^7$),
7.83 (d, 2H, J = 9.2 Hz, aromatic),
7.96 (d, 1H, J = 2.0 Hz, $H^5$),
12.22 (br s, 1H, NH).
Anal. ($C_{20}H_{16}F_3N_3O_3S$) C,H,N,F.

(ii) N-((3,4-Dihydro-2-methyl-4-oxo-6-quinazolinyl)methyl)-4-(phenylsulfonyl)-N-(prop-2-ynyl)-3-(trifluoromethyl)aniline. (Compound B in Tables III and IV below.)

The above procedure was repeated starting from 4-(phenylsulfonyl)-N-(prop-2-ynyl)-3-(trifluoromethyl)-aniline (1.27 g, 3.74 mmol) in DMF (20 mL)) with reagents in the following quantities: 60% sodium hydride (0.15 g, 3.70 mmol), the bromomethylquinazoline (1.36 g, 3.70 mmol) in DMF (20 mL), $NaHCO_3$ aq (400 mL), MeOH (90 mL)/1N LiOH (9 mL, 9 mmol). Exhaustive extraction with $CH_2Cl_2$ (11 x 100 mL) was needed to obtain the crude product as an amber oil (1.37 g). This was flash chromatographed (50% $CH_3CN$ in $CCl_4$) to give the pure product as an off-white solid (0.207 g, 10.9%) mp 235.5-237°C. NMR satisfactory. Anal. ($C_{26}H_{20}F_3N_3O_3S$) C,H,N,F,S.

(iii) N-((3,4-dihydro-4-oxo-2-phenyl-6-quinazolinyl)methyl)-N-prop-2-ynyl)-4-chloroaniline. (Compound C in Table III below.)

In a flame-dried flask equipped with a magnetic stir bar was placed 6-bromomethyl-3,4-dihydro-4-oxo-2-phenyl-quinazoline (European Patent Application Number 87302525.8, published September 30, 1987) (0.309 g, 0.95 mmol), 4-chloro-N-propargylaniline (0.158 g, 0.95 mmol), sodium iodide (0.143 g, 0.95 mmol), $K_2CO_3$ (0.263 g, 1.90 mmol) and DMA (10 mL) while under an argon atmosphere. The reaction mixture was gradually heated to 90°C and maintained for 3 h. The mixture was then cooled to room temperature and slowly poured into $H_2O$ (150 mL). A solid precipitated which was filtered off and washed with cold $H_2O$ (25 mL) and then diethyl ether (50 mL). The solid was dried over $P_2O_5$ in vacuo overnight and purified by flash

column chromatography (3% $CH_3CN$ in $CH_2Cl_2$). Evaporation of the appropriate fractions gave the pure product as a white powder (0.161 g, 43%), mp 247-252° C, $R_f$=0.15 (silica: 2% $CH_3CN$ in $CH_2CL_2$).

$^1$H NMR ($Me_2SO-d_6$)delta:
3.23 (t, 1H, J = 2.2 Hz, :::CH)
4.26 (d, 2H, J = 2.2 Hz, $CH_2$-C:::)
4.70 (s, 2H, N-$CH_2$-Ph)
6.84 (d, 2H, J = 9.1 Hz, aromatic)
7.21 (d, 2H, J = 9.1 Hz, aromatic)
7.56 (m, 5H, aromatic)
7.74 (d, 1H, J = 7.9 Hz, $H^8$)
8.04 (d, 1H, J = 1.6 Hz, $H^5$)
8.17 (dd, 1H, J = 7.9 & 1.6 Hz, $H^7$)
12.54 (br s, 1H, NH)
Anal. ($C_{24}H_{18}ClN_3O$) C,H,N,Cl.

(iv) N-((3,4-dihydro-2-methyl-4-thio-6-quinazolinyl)methyl)-N-(prop-2-ynyl)-3-trifluoromethylaniline. (Compound D in Table III below.)

In a flame-dried two necked flask equipped with a reflux condensor and magnetic stir bar was placed N-((3,4-dihydro-3-methyl-4-oxo-6-quinazolinyl)methyl-N-(prop-2-ynyl)-3-trifluoromethylaniline (0.371 g, 1.0 mmol), $CH_3CN$ (4 mL), $Et_3N$ (0.56 mL, 4.0 mmol), and $P_2S_5$ (0.445 g, 2.0 mmol) while under an argon atmosphere. The mixture was heated to 50° C whereupon most of the $P_2S_5$ dissolved. Heating was continued overnight. The mixture was cooled and poured into $H_2O$ (100 mL). A solid precipitated which was filtered off and washed with cold $H_2O$. The solid was dried over $P_2O_5$ in vacuo and purified by flash column chromatography (2% $CH_3CN$ in $CH_2Cl_2$). Evaporation of the appropriate fractions gave a pale yellow solid (0.368 g, 95%) mp 232-234° C, $R_f$ = 0.34 (silica: 5% $CH_3CN$ in $CH_2Cl_2$).

$^1$H NMR ($Me_2SO-d_6$)delta:
2.44 (s, 3H, $CH_3$)
3.24 (t, 1H, J = 2.1 Hz :::CH)
4.33 (d, 2H, J = 2.1 Hz, $CH_2$-C:::)
4.79 (s, 2H, N-$CH_2$-Ph)
7.00-7.1 (m, 3H, aromatic)
7.39 (t, 1H, J = 8.25 Hz, aromatic)
7.61 (d, 1H, J = 8.4 Hz, $H^8$)
7.76 (dd, 1H, J = 8.4 & 2.0 HZ, $H^7$)
8.48 (d, 1H, J = 2.0 Hz, $H^5$)
13.68 (br, s, 1H, NH).
Anal. ($C_{20}H_{16}F_3N_3S$) C,H,N,S.

The following is a procedure for preparing a compound of the invention where $R_L$ is a benzofuran ring system.

2-Hydroxy-3-methylbenzonitrile

To a rapidly stirred solution of 3-methylsalicylic acid (5.0 g, 32.9 mmol) in benzene (150 mL) was added at room temperature during 5 min. oxalyl chloride (3.15 mL, 36 mmol) via a syringe followed by 50 microliters of DMF. After 2 h. anhydrous ammonia was bubbled into the reaction mixture for 30 min. after which the whole was poured into water (300 mL) and brought to pH 7 with 6N HCl. The aqueous layer was extracted with $CH_2Cl_2$ (3 x 150 mL), and the combined organic layers were dried ($Na_2SO_4$). The solvent was removed under reduced pressure to give 5.0 g (100%) of crude 2-hydroxy-3-methylbenzamide as a yellow solid. This material was used directly in the next reaction. A solution of the material (2.0 g, 13.2 mmol) in THF (40 mL) at 0° C containing pyridine (4.5 mL, 55 mmol) was cooled to 0° C, and trifluoroacetic anhydride (4.5 mL, 31.7 mmol) was added during 10 min. After the addition was complete, the mixture was allowed to warm to room temperature for 3 hr. and then poured into saturated $NaHCO_3$ (100 mL). The aqueous layer was extracted with $CH_2Cl_2$ (3 x 150 mL), and the combined organic layers were dried ($Na_2SO_4$). The solvent was removed under reduced pressure, and the residue was flash chromatographed on silica (200 g) with 20% EtOAc in petrol. In this manner, there was obtained the nitrile (1.47 g, 84%) as a

white solid, mp 85-87° C which is consistent with the literature value (British Patent 1,090,155).

2-(Ethoxycarbonyl)methoxy-3-methylbenzonitrile

To a rapidly stirred solution of 2-hydroxy-3-methylbenzonitrile (50 mg, 0.37 mmol) in acetone (2 mL) at room temperature was added $K_2CO_3$ (104 mg, 0.75 mmol) all at once followed by ethyl bromoacetate (46 microliters, 0.41 mmol) during 5 min. The resulting mixture was heated to 72° C for 1h and then poured into water (20 mL) and brought to pH 2 with 6N HCl. The aqueous layer was extracted with $CH_2Cl_2$ (3 x 50 mL) and dried ($Na_2SO_4$). The solvent was removed under reduced pressure and the residue was flash chromatographed on silica (10 g) with 18% EtOAc in petrol to give the desired product as a colorless oil (86 mg, 100%).

NMR ($CDCl_3$)delta:

1.29 (t, 3H, J=7.1Hz, $CH_3$), 2.34 (s, 3H, $CH_3$), 4.25 (q, 2H, J=7.1Hz, O-$CH_2$), 4.77 (s, 2H, O-$CH_2$), 7.07 (t, 1H, J=7.7Hz), 7.40 (br d, 2H, J=7.7Hz).

IR (neat) $cm^{-1}$; 2985 (s), 2938 (m), 2230 (m, C:::N), 1750 (s, C=O), 1585 (w), 1460 (s), 1380 (m). Anal. ($C_{12}H_{13}NO_3$) C,H,N.

Ethyl 3-amino-7-methylbenzofuran-2-carboxylate

To a rapidly stirred solution of 2-(ethoxycarbonyl) methoxy-3-methylbenzonitrile (1.9 g, 8.67 mmol) in DMF (30 mL) at room temperature was added $K_2CO_3$ (1.92 g, 13.9 mmol) all at once. The resulting mixture was heated to 150° C for 30 min. and then poured into water (150 mL). The mixture was acidified to pH 2 with 6N HCl, extracted with ether (2 x 150 mL), and the combined organic layers were dried ($Na_2SO_4$). The solvent was removed under reduced pressure, and the residue was flash chromatographed on silica (200 g) to give the desired product as an off white solid, (1.84 g, 97%) mp 118-119.5° C.

NMR ($CDCl_3$)delta:

1.44 (t, 3H, J=7.1Hz, $CH_3$), 2.52 (s, 3H, $CH_3$), 4.43 (q, 2H, J=7.1Hz, O-$CH_2$), 7.14 (t, 1H, J=7.6Hz), 7.24 (d, 1H, J=7.6Hz), 7.37 (d, 1H, J=7.6Hz).

IR (KBr) $cm^{-1}$; 3495 (m), 3320 (m), 1665 (s, C=O), 1625 (s), 1575 (m), 1440 (m), 1295 (m). Anal. ($C_{12}H_{13}NO_3$) C,H,N.

Ethyl 3-formylamino-7-methylbenzofuran-2-carboxylate

To a rapidly stirred solution of ethyl 3-amino-7-methylbenzofuran-2-carboxylate (1.72 g, 7.85 mmol) in $CH_2Cl_2$ (50 mL) at room temperature was added 98% formic acid (0.6 mL, 15.7 mmol) followed by dicyclohexylcarbodiimide (2.1 g, 10.2 mmol) as a solid. After 2h., the mixture was poured into half-saturated $NaHCO_3$ (200 mL) and the mixture was extracted with EtOAc (3 x 200 mL) and the combined organic layers were dried ($Na_2SO_4$). The solvent was removed at reduced pressure and the residue was flash chromatographed on silica (150 g) with 2% EtOAc in petrol to give the desired formamide as a white solid, (1.94 g, 99%) mp 162-164° C.

NMR ($CDCl_3$) delta:

1.48 (t, 3H, J=7.2Hz, $CH_3$), 2.55 and 2.58 (s, 3H, -$CH_3$), 4.50 (q, 2H, J=7.2Hz, O-$CH_2$), 7.25 (m, 2H), 7.53 and 8.26 (d, 1H, J=8Hz), 8.48 (s, .5H, O=C-H), 9.06 (m, .5H, NH), 9.23 (d, .5H, J=11.5Hz, O=C-H), 9.42 (m, .5H, NH).

IR (KBr) $cm^{-1}$: 3275 (s), 2995 (w), 2905 (w), 1710 (s), 1660 (s), 1620 (s), 1500 (w), 1380 (m), 1345 (w), 1295 (s), 1210 (m). Anal. ($C_{13}H_{13}NO_4$) C,H,N.

Ethyl 3-formylamino-7-(bromomethyl)benzofuran-2-carboxylate

To a rapidly stirred solution of ethyl 3-formylamino-7-methylbenzofuran-2-carboxylate (0.5 g, 2.02 mmol), in $CCl_4$ (25 mL) at room temperature was added N-bromosuccinimide (395 mg, 2.22 mmol) all at once. The resulting mixture was heated to reflux and irradiated with a 500W incandescent light for 10 min. and then poured into water (100 mL). The mixture was extracted with $CH_2Cl_2$ (3 x 100 mL) and the combined organic layers were dried ($Na_2SO_4$). The solvent was removed under reduced pressure and the

residue was flash chromatographed on silica gel (50 g) with 2% ÈtOAc in $CH_2Cl_2$ to give the desired bromide as a white solid, (448 mg, 68%) mp 202-204 ° C.

NMR ($CDCl_3$) delta:

1.46 (t, 3H, J=7.1Hz, $CH_3$), 4.48 (q, 2H, J=7.1Hz, O-$CH_2$), 4.79 (s, 2H, $CH_2Br$), 7.27 (m, 1H), 7.55 (m, 1H), 8.42 (d, 1H, J=7.5Hz), 8.46 (s, .5H, O=C-H), 9.02 (m, .5H, NH), 9.20 (d, .5H, J=12Hz, O=C-H), 9.43 (m, .5H, NH).

IR (KBr) $cm^{-1}$: 3280 (m), 2995 (w), 2890 (w), 1705 (s), 1670 (s), 1615 (s), 1500 (w), 1380 (w), 1345 (w), 1295 (s), 1215 (m). Anal. ($C_{13}H_{12}BrNO_4$) C,H,N,Br.

N-((2-Carboxyethyl-3-formylamino-7-benzofuranyl)methyl)-N-(prop-2-ynyl)-4-nitroaniline.

To a rapidly stirred solution of ethyl 3-formylamino-7-(bromomethyl)benzofuran-2-carboxylate (366 mg, 1.12 mmol) in DMF (9 mL) at room temperature was added 4-nitro-N-propargylaniline (217 mg, 1.23 mmol) and $CaCO_3$ (235 mg, 2.35 mmol). The resulting mixture was heated at 130 ° C for 3h. and then poured into water (50 mL). The mixture was extracted with $CH_2Cl_2$ (3 x 100 mL) and the combined organic layers were dried ($Na_2SO_4$). The solvent was removed under reduced pressure, and the residue was flash chromatographed on silica (50 g) with 3% EtOAc in $CH_2Cl_2$ to give the desired product as a yellow solid (238 mg, 50%) mp 203-206 ° C.

NMR ($CDCl_3$) delta:

1.44 (t, 3H, J=7.1Hz, $CH_3$), 2.32 (m, 1H, :::CH), 4.26 (m, 2H, $CH_2$-C:::), 4.46 (q, 2H, J=7.1Hz, O-$CH_2$), 5.00 and 5.04 (s, 1H, Ar$CH_2$-N), 6.86 (d, 2H, J=9.4Hz), 7.3 (m, 2H), 8.12 (d, 2H, J=9.4Hz), 8.41 (d, 1H, J=8Hz), 8.47 (s, .8H, O=C-H), 9.02 (m, .2H, NH), 9.21 (d, .2H, J=11Hz, O=C-H), 9.39 (brs, .8H, NH).

IR (KBr) $cm^{-1}$: 3295 (m), 2995 (w), 2915 (w), 1700 (s), 1590 (s), 1480 (m), 1320 (s). Anal. ($C_{22}H_{19}N_3O_6$) C,H,N.

N-((3-Formylamino-7-benzofuranyl)methyl)-N-(prop-2-ynyl)-4-nitroaniline. (Compound E in Table III.)

To a rapidly stirred solution of N-((2-carboxyethyl-3-formylamino-7-benzofuranyl)methyl)-N-(prop-2-ynyl)-4-nitroaniline (228 mg, 0.54 mmol) in DMSO (4 mL) at room temperature was added LiCl (27 mg, 0.65 mmol) and $H_2O$ (20 microliters, 1.1 mmol). The resulting mixture was heated to 170 ° C for 8h, and then poured into half-saturated $NaHCO_3$ (50 mL) and extracted with EtOAc (3 x 60 mL) and the combined organic layers were then washed with $H_2O$ (30 mL) and dried ($Na_2SO_4$). The solvent was removed under reduced pressure and the residue was chromatographed on silica (Merck, Art 7734) (20 g) with 8% EtOAc in $CH_2Cl_2$ to give the desired product as a yellow solid (90 mg, 48%), mp 188-190 ° C.

NMR ($CDCl_3$) delta:

2.32 (m, 1H, :::CH), 4.26 (d, 2H, J=2.4Hz, $CH_2$-C:::), 4.99 (s, 2H, Ar$CH_2$N), 6.82 (d, 2H, J=9.6Hz), 7.2 (m, 2H), 7.45 (d, 1H, J = 6.4 Hz), 7.59 (s, .5 H, NH), 8.11 (d, 2H, J = 9.6 Hz), 8.41 (s, .5H, O=C-H), 8.52 (s, 0.5H, O=C-H).

IR(KBr) $cm^{-1}$: 3300 (m), 1680 (m), 1595 (s), 1310 (s). High Resolution Mass Spec. for ($C_{19}H_{15}N_3O_4$). Calc 349.1062; found 349.1069.

The following Table I describes the preparation of suitable starting materials for use in the invention.

Table I. Preparation of Propargylanilnes

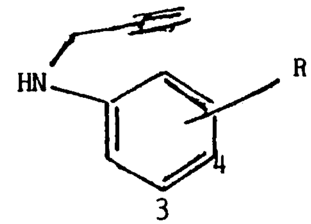

| Substituents $R^3$ and $R^4$ | Quantity of Reactant (mmol) Ani- line | Prop- argyl bro- mide | $K_2CO_3$ | Sol- vent | mL | Reac- tion temp, °C | Reac- tion time, h. | Chroma- tography[a] | Yield % | Form | Formula | Anal. |
|---|---|---|---|---|---|---|---|---|---|---|---|---|
| — | 200 | 100 | 100 | MeOH | 100 | 25 | 48 | 5% EP | 73 | oil | $C_9H_9N$ | b |
| $3-CH_3$ | 100 | 100 | 100 | MeOH | 100 | 25 | 2 | 10% EP | 36 | oil | $C_{10}H_{11}N$ | C,H,N |
| $3-CF_3$ | 100 | 100 | 100 | MeOH | 100 | 65 | 3.25 | 10% EP | 22 | oil | $C_{10}H_8F_3N$ | C,H,N,F |
| $3-C_2H_5$ | 83 | 66 | 79 | MeOH | 100 | 43 | 1.33 | 10% EP | 41 | oil | $C_{11}H_{13}N$ | C,H,N |
| 3-Br | 58 | 58 | 58 | MeOH | 100 | 59 | 2.75 | 8% EP | 21 | oil | $C_9H_8BrN$ | C,H,N,Br |
| 4-CN | 68 | 102 | 68 | DMF | 50 | 95 | 3.66 | 40% EP | 29 | solid[c] | $C_{10}H_8N_2$ | C,H,N |
| 3-I | 50 | 50 | 50 | EtOH | 100 | 50 | 3 | 10% EP | 23 | oil | $C_9H_8IN$ | C,H,N,I |
| 3-Cl,4-CN | 50 | 50 | 50 | DMF | 50 | 105 | 5 | 60% EP | 28 | solid[d] | $C_{10}H_7ClN_2$ | C,H,N |
| 3-C≡CH | 43 | 51 | 43 | MeOH | 40 | 25 | 22 | " | 38 | oil | $C_{11}H_9N$ | C,H,N |
| $3-CF_3,4-NO_2$ | 30 | 30 | 30 | DMF | 50 | 105 | 6.5 | 60% $CH_2Cl_2$ in petrol[f] | 16 | solid[g] | $C_{10}H_7F_3N_2O_2$ | C,H,N,F |
| $4-COC_6H_5$ | 50 | 60 | 50 | DMA | 50 | 110 | 1.66 | 40% EP[h] | 30 | solid[i] | $C_{16}H_{13}NO$ | C,H,N |
| $3-CF_3,4-CN$ | 40 | 50 | 40 | DMA | 50 | 100 | 11.66 | 60% $CH_2Cl_2$ in petrol[j] | 43 | solid[k] | $C_{11}H_7F_3N_2$ | C,H,N,F |

| | | | | | | | | | | | |
|---|---|---|---|---|---|---|---|---|---|---|---|
| 4-$SO_2C_6H_5$ | 34 | 44 | 34 | DMA | 50 | 100 | 3.50 | 10% $CH_3OH$ in toluene[l] | 55 | solid[m] | $C_{15}H_{13}NO_2S$ | C,H,N,S |
| 4-$SO_2NH_2$[n] | 50 | 50 | 50[o] | DMA | 50 | 100 | 2 | 15% $CH_3CN$ in $CH_2Cl_2$ | 31 | solid[p] | $C_9H_{10}N_2O_2S$ | C,H,N,S |
| 3-$CF_3$, 4-$SO_2C_6H_5$ | 19 | 57 | 19 | DMF | 20 | 110 | 4 | 1% $CH_3CN$ in $CH_2Cl_2$ | 21 | solid[q] | $C_{16}H_{12}F_3NO_2S$ | C,H,N,F,S |
| 3-$CF_3$, 4-$SO_2NH_2$[r] | 20 | 24 | 20[o] | DMA | 25 | 100 | 3 | 10% $CH_3CN$ in $CH_2Cl_2$ | 57 | solid[s] | $C_{10}H_9F_3N_2O_2S$ | C,H,N,F,S |
| 3-$CF_3$, 4-$SO_2NH$-$CH_2COOCH_3$[r] | 10 | 35 | 10[o] | DMA | 20 | 105 | 3.66 | 7% $CH_3CN$ in $CH_2Cl_2$ | 17 | solid[t] | $C_{13}H_{13}F_3N_2O_4S$ | C,H,N,F,S |

[a] x%EP = x% ether in petrol.

[b] Lit: J.v. Braun and L. Tauber, Justus Liebig's Ann. Chem. 458, 102 (1927).

[c] White flakes ($CCl_4$) mp 65-66°C.

[d] Pale yellow microneedles (EtOH) mp 127-128°C.

[e] Eluted sequentially with hexane, 1%, 2%, and finally 4% $CH_2Cl_2$ in hexane.

[f] A strange by-product eluted last and characterized as a yellow oil (2.56g, 33%) which NMR and microanalysis (C,H,N) proved to be $N^1,N^1$-dimethyl-$N^3$-(4-nitro-3(trifluoromethyl)phenyl)formamidine.

[g] Yellow-orange needles (EtOH, $H_2O$) mp 133-134 °C.

[h] The crude product was coated onto silica (Merck Art 7734, 40g) from $CH_3CN$/ether solution and applied to the column thus.

[i] Yellow plates (benzene/hexane) mp 152.5-153.5°C.

[j] Crude product applied dissolved in this solvent mixture containing some (0.5mL) DMF.

[k] White microneedles ($EtOH/H_2O$) mp 131-132°C.

[l] The crude product was coated onto silica (Merck Art 7734, 24g) from ether/acetone and applied to the column thus.

[m] Pale yellow needles (benzene/hexane) mp 97-98°C. An alternative procedure for obtaining this amine is described above.

[n] $CH_2Cl_2$ was used in the work-up in place of ether.

[o] $CaCO_3$ was used in place of $K_2CO_3$ and was filtered off on celite before work-up.

[p] Pale yellow plates ($CH_3CN/CCl_4$) mp 131.5-132°C.

[q] Off-white needles (benzene/hexane) mp 131-132°C.

[r] EtOAc was used in the work-up in place of ether.

[s] Beige plates (benzene/hexane) mp 125-126°C.

[t] Beige plates (benzene/hexane) mp 124-125°C.

The following Table II describes the preparation of quinazolines of formula (I) of the invention.

TABLE II. Preparation of Quinazolines of the Invention

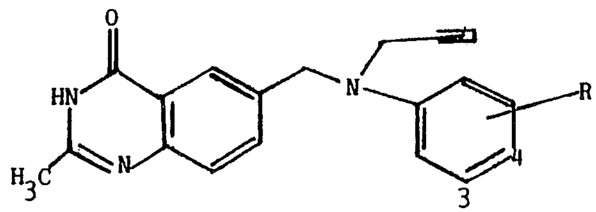

| Substituents $R^3$ and $R^4$ | Quantity of Reactant (mmol) Aniline | Quinazoline | $CaCO_3$ | Solvent | mL | Reaction temp, °C | Reaction time, h. | Purif | Yield % | mp °C | Formula | Anal. |
|---|---|---|---|---|---|---|---|---|---|---|---|---|
| - | 7.9 | 7.9 | 7.9 | DMF | 30 | 95 | 2 | a | 33 | 242.5-243.5 | $C_{19}H_{17}N_3O$ | C,H,N |
| 4-Cl | 20 | 20 | 40 | DMA | 40 | 95 | 2 | b | 65 | 240-240.5 | $C_{19}H_{16}ClN_3O$ | C,H,N,Cl |
| $3-CH_3$ | 10 | 10 | 20 | DMF | 30 | 95 | 2 | c | 44 | 229-230.5 dec | $C_{20}H_{19}N_3O$ | C,H,N |
| $3-CF_3$ | 10 | 10 | 20 | DMF | 30 | 100 | 1.8 | d | 32 | 228-229.5 | $C_{20}H_{16}F_3N_3O$ | C,H,N,F |
| $3-C_2H_5$ | 10 | 10 | 20 | DMF | 20 | 105 | 1.8 | e | 33 | 195.5-197 | $C_{21}H_{21}N_3O$ | C,H,N |
| 3-Br | 9.5 | 9.5 | 19 | DMF | 30 | 110 | 1.8 | f | 32 | 227.5-229 dec | $C_{19}H_{16}BrN_3O$ | C,H,N,Br |
| $3-CH_2OH$ | 4.65 | 4.65 | 4.65[g] | DMF | 25 | 90 | 2 | h | 58 | 215-217 | $C_{20}H_{19}N_3O_2$ | C,H,N |
| $3-CH(OH)CH_3$ | 5.71 | 5.71 | 5.71[g] | DMF | 25 | 105 | 2 | i | 46 | 171-180 | $C_{21}H_{21}N_3O_2$ | C,H,N |
| 4-CN | 10 | 10 | 20 | DMF | 30 | 110 | 3 | j | 13[k] | 266-267.5 dec | $C_{20}H_{16}N_4O$ | C,H,N |
| 3-I | 5 | 5 | 10 | DMF | 30 | 100 | 2 | l | 34 | 249.5-250.5 | $C_{19}H_{16}IN_3O$ | C,H,N,I |
| 3-Cl,4-CN | 10 | 10 | 20 | DMF | 30 | 110 | 3.6 | m | 18 | 267-268 | $C_{20}H_{15}ClN_4O$ | C,H,N,Cl |

| | | | | | | | | | | | | |
|---|---|---|---|---|---|---|---|---|---|---|---|---|
| 4-$NO_2$ | 10 | 10 | 20 | DMF | 30 | 110 | 3.3 | n | 20 | 278-279 dec | $C_{19}H_{16}N_4O_3$ | C,H,N |
| 3-C:::CH | 3.22 | 3.22 | 6.44 | DMA | 15 | 80 | 3 | o | 29 | 234-236 dec | $C_{21}H_{17}N_3O$ | C,H,N |
| 3-$CF_3$ 4-$NO_2$ | 3.85 | 3.85 | 7.7 | DMF | 50 | 115 | 24 | p | 2.3 | 261-262 dec | $C_{20}H_{15}F_3N_4O_3$ | q |
| 4-COPh | 10 | 10 | 20 | DMA | 35 | 115 | 3.5 | r | 23 | 213-214.5 dec | $C_{26}H_{21}N_3O_2$ | C,H,N |
| 3-$CF_3$ 4-CN | 10 | 10 | 20 | DMA | 30 | 115 | 6 | s | 5 | 270-271.5 | $C_{21}H_{15}F_3N_4O$ | C,H,N,F |
| 4-$SO_2$Ph | 10 | 10 | 20 | DMA | 30 | 115 | 4 | t | 13 | 191-193 | $C_{25}H_{21}N_3O_3S$ | C,H,N,S |
| 4-$SO_2NH_2$ | 10 | 10 | 20 | DMA | 30 | 105 | 2 | u | 35 | dec over 278 | $C_{19}H_{18}N_4O_3S$ | C,H,N,S |
| 3-$CF_3$ 4-$SO_2NH_2$ | 10 | 10 | 20 | DNA | 30 | 90 | 3.5 | v | 21 | dec over 286 | $C_{20}H_{17}F_3N_4O_3S$ | C,H,N,F,S |
| 3-$CF_3$ 4-$SO_2$NH-$CH_2$COOH[w] | 16 | 32 | 32 | DNA | 25 | 110 | 9 | x | 19 | 247.5-248.5 dec | $C_{23}H_{21}F_3N_4O_5S$ | C,H,N,S |

[a] 1.87 g recrystallized from acetone to give off-white microneedles.

[b] 6.01 g twice recrystallized from 1,2-dichloroethane to give white microneedles.

[c] 2.62 g twice recrystallized from EtOAc to give off-white microneedles.

[d] 2.82 g twice recrystallized from EtOAc to give off-white microneedles.

[e] 2.63 g twice recrystallized from EtOAc to give white microneedles.

[f] 3.00 g twice recrystallized from EtOAc to give off-white microneedles.

[g] $K_2CO_3$ was used in place of $CaCO_3$ and the celite filtration omitted.

[h] 1.09 g taken up in hot MeOH (150mL); the filtered, cooled solution was diluted with $H_2O$ (250mL) to precipitate the pure product as a beige crystalline solid.

[i] 1.37 g taken up in hot MeOH (100mL); the filtered, cooled solution was diluted with $H_2O$ (250mL) to precipitate the pure product as a beige crystalline solid.

[j] 2.03 g flash chromatographed with 40% $CH_3CN$ in $CH_2Cl_2$ to give a white solid; the sample was applied in the eluant with the aid of a little DMF.

[k] Not optimized.

[l] 2.07 g twice recrystallized from EtOAc to give white crystals.

[m] Extracted with $CHCl_3$ to give 3.03 g which was coated onto $SiO_2$ from $CHCl_3$ and flash chromatographed using 40% $CH_3CN$ in $CH_2Cl_2$ as eluant to give a white solid.

[n] 2.78 g coated onto $SiO_2$ from $CHCl_3$ and flash chromatographed eluting sequentially with $CH_3CN$, and 15% MeOH in $CH_3CN$ to give a yellow solid.

[o] 0.42 g flash chromatographed eluting sequentially with 90% $CH_2Cl_2$ in hexane, $CH_2Cl_2$, and 1.5% $CH_3CN$ in $CH_2Cl_2$ to provide white flakes.

[p] Extracted with EtOAc to give 0.65 g which was flash chromatographed using 40% $CH_3CN$ in $CH_2Cl_2$ to give 0.1 g of material 84% pure by HPLC. This was coated onto $SiO_2$ from $CH_3CN$ and re-chromatographed using 30% ether in EtOAc to give a yellow solid 98% pure by HPLC.

[q] This compound failed to microanalyze well. C: calcd, 57.70; found 58.83, 58.62. H: calcd, 3.63; found 4.17, 4.18. N: calcd, 13.46; found 12.46, 12.54. High resolution mass spectrometry proved the structure. Calcd for $C_{20}H_{15}F_3N_4O_3$ 416.1096; found 416.1100

[r] 3.81 g twice recrystallized from 1,2-dichloroethane to give off-white microneedles.

[s] Extracted with EtOAc to give 1.96 g which was coated onto $SiO_2$ from $CH_3CN$ and flash chromatographed using $CH_3CN$ as eluant to give a white solid. The analytical sample was recrystallized from $CH_3CN$.

[t] 3.83 g coated onto $SiO_2$ from 1,2-dichloroethane and flash chromatographed using 10% MeOH in benzene to give 2.19 g of impure material which was re-chromatographed using 5% MeOH in $CH_2Cl_2$ to give 0.56 g which was recrystallized from benzene/hexane to give off-white crystals.

[u] 2.94 g coated onto $SiO_2$ from DMF and flash chromatographed using 5% MeOH/EtOAc to give a white solid.

[v] 3.05 g coated onto $SiO_2$ from DMF and flash chromatographed using 80% $CH_3CN$ in $CH_2Cl_2$ to give a white solid.

[w] This entry describes the preparation of the corresponding methyl glycinate ester Saponification of this ester to give the carboxylic acid derivative, as listed under R, is given in footnote y.

[x] 0.40 g coated onto $SiO_2$ from DMF and flash chromatographed using 80% $CH_3CN$ in $CCl_4$ to give a white solid.

[y] N-($N^4$-((3,4-dihydro-2-methyl-4-oxo-6-quinazolinyl)methyl)-$N^4$-(prp-2ynyl)-2-(trifluormethyl)-sulfanilyl)glycine.

The methylglycinate ester (last entry in Table II) (0.138 g, 0.264 mmol) was suspended in a 1:1 mixture of THF:$H_2O$ (8 mL) and treated with 1N L:OH (1.32 mL, 1.32 mmol) to give a solution which was kept at 20°C for 3h. The solution was filtered and brought to pH 2.0 with 1N HCl to produce a gelatinous white precipitate. This was centrifuged and washed by three cycles of resuspension ($H_2O$, 15 mL)-centrifugation-decantation. The product was dried over $P_2O_5$ in vacuo at 20°C and was obtained as an amorphous white solid (0.087g, 65%), mp 237°C dec. NMR ($Me_2SO$-$d_6$) satisfactory. Anal..($C_{22}H_{19}F_3N_4O_5S$) C,H,N.

Determination of Inhibition Constants Against 5,10-Methylene-Tetrahydrofolate

Thymidylate synthase inhibition constants $K_1$ have been determined by the following method. All assays were run at 25° C and initiated by the addition of thymidylate synthase ("TS"). TS has ordered bireactant kinetics [Daron, H.H. and Aull, J.L. (1978), J. Biol. Chem. 253, 940-945], and the dUMP(2′-deoxyuridine-5′-monophosphate) concentration used for these reactions was near saturation levels so that the assays were pseudo-single substrate. All reaction mixtures contained 50 mM Tris at pH 7.8 (the final pH of the reaction was 7.6), 10 mM DTT (dithiothreitol), 1 mM EDTA (ethylenediaminetetraacetic acid), 25 mM $MgCl_2$, 15 mM $H_2CO$ (formaldehyde) and 25 microM dUMP. When human or Leishmania TS was assayed, 100 micrograms/ml of BSA (bovine serum albumin) was present in the reactions. The range of THF (tetrahydrofolate) was 5 to 150 microM (eight concentrations: 5, 6.6, 10, 13, 16, 25, 50 and 150 microM. A standard curve in the absence of inhibitor was run with each experiment. Three curves were then run with inhibitor at three different concentrations with a minimal range, where possible, from 1/2 to 2 times the $K_i$ - (Cleland, W.W. (1963) Biochim. Biophys. Acta 67, 173-187]. These assays were done on a spectrophotometer at 340 nm [Wahba, A.J. and Friedkin, M. (1961) J. Biol. Chem. 236, PC11-PC12] (following the formation of DHF (dihydrofolate); mM extinction coefficient of 6.4) or by following the release of tritium [Lomax, M.I.S. and Greenberg, G.R. (1967) 242, 109-113] from the 5 position of dUMP (assays for tritium release contained 0.5 micro (l dUMP). Charcoal was used to remove unreacted dUMP from the tritium release reaction mixtures and the resultant water was counted to determine the extent of reaction. Inhibition constants were then determined by plotting the apparent $K_m$ or the reciprocal of the apparent $V_{max}$ against the inhibitor concentration [Cleland, W.W. (1970) The Enzymes 2, 1-65].

The results obtained are tabulated below in Table III.

TABLE III

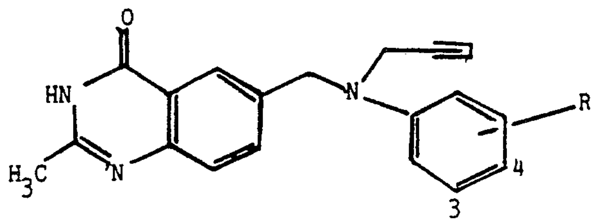

| R | $K_iTS$ (µM) | | | |
|---|---|---|---|---|
| | E.coli | Human | C.albicans | L. major |
| 4-Cl | 5.9 ± 1.8 | 5.4 | 10.6 ± 4.7 | |
| 3-$CH_3$ | 16.3 ± 9.8 | 10.2 ± 10.2 | 7.7 | |
| 3-$CF_3$ | 1.1 ± 0.7 | 1.3 ± 0.7 | 3.8 ± 1.3 | 3.4 ± 0.2 |
| — | 13.9 ± 7.3 | 8.4 ± 4.6 | 19.0 ± 15.9 | 16.9 ± 4.0 |
| 3-$C_2H_3$ | 24.5 ± 15.7 | 23.9 ± 20.8 | | |
| 3-Br | 5.5 ± 1.3 | 3.6 ± 2.6 | | |
| 3-$CH_2OH$ | 6.7 ± 5.4 | 1.2 ± 0.4 | | |
| 3-$CH(OH)CH_3$ | 11.4 ± 9.8 | 9.6 ± 6.9 | | |
| 4-CN | 0.52 ± 0.46 | 0.54 ± 0.74 | | |
| 3-I | 49.0 ± 42.3 | 4.7 ± 3.7 | 15.5 ± 5.5 | |
| 3-Cl, 4-CN | 0.091 ± 0.052 | 0.087 ± 0.001 | 0.28 ± 0.19 | |
| 4-$NO_2$ | 0.12 ± 0.07 | 0.25 ± 0.21 | 1.5 ± 1.1 | |
| 3-C≡CH | 32.9 ± 14.1 | | | |
| 3-$CF_3$,4-$NO_2$ | 0.078 ± 0.076 | 0.20 ± 0.25 | | |
| 4-COPh | 0.50 ± 0.11 | 0.70 ± 0.19 | | |
| 3-$CF_3$,4-CN | 0.036 ± 0.007 | 0.081 ± 0.057 | | 0.030 |
| 4-$SO_2Ph$ | 0.11 ± 0.09 | 0.029 ± 0.025 | 0.68 ± 0.44 | 0.037 |
| 4-$SO_2NH_2$ | 0.25 ± 0.15 | 0.16 ± 0.12 | 1.0 | |
| 3-$CF_3$,4-$SO_2NH_2$ | 0.19 ± 0.12 | 0.58 ± 0.74 | 1.9 ± 0.6 | |
| 3-$CF_3$,4-$SO_2NHCH_2COOH$ | 0.015 ± 0.003 | | | 0.013 |

TABLE III (Cont'd)

| | $K_1$TS (μM) | | | |
|---|---|---|---|---|
| | E.coli | Human | C.albicans | L. major |
| Compound A* | 0.015 ± 0.058 | 0.075 ± 0.035 | | |
| Compound B | 0.061 ± 0.027 | 0.13 ± 0.06 | 2.6 ± 2.8 | |
| Compound C | 42.6 ± 23.1 | | | |
| Compound D | 3.3 ± 1.4 | 17.5 ± 11.0 | | |
| Compound E | 70.9 ± 1.8 | | | |

In Vitro Testing

Cellular growth in the presence of the compounds in question was assessed using two cell lines, the LI2I0 murine leukemia (ATCC CCL 219) and CCRF-CEM, a human lymphoblastic leukemia line of T-cell origin (ATCC CCL 119). Both lines were maintained in RPMI 1640 medium containing 5% heat-inactivated fetal bovine serum without antibiotics.

$IC_{50}$ values were determined in 150 microliter microcultures each containing 1500 (LI2I0) or 4000 (CCRF-CEM) cells established in 96 well plates in growth medium supplemented with 50 IU/ml penicillin and 50 mcg/ml streptomycin. Growth was measured over 3 days (LI2I0) or 5 days (CCRF-CEM) of continuous exposure to varying concentrations of each test compound added 4 h. after initial cell plating by the MTT-tetrazolium reduction assay of Mosmann (T. J. Immunol. Meth. 65, 55-63 (1983)) modified according to Alley et al. (Cancer Res. 48 589-601 (1988)). Water insoluble derivatives were dissolved in DMSO and diluted to a final concentration of 0.5% solvent in cell cultures.

The results obtained from this procedure are tabulated below in Table IV:

TABLE IV

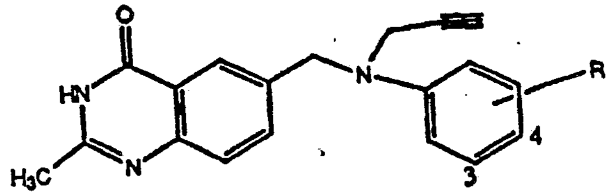

IC$_{50}$ FOR CELLS IN CULTURE

| Substituents $R^3$ and $R^4$ | L1210 | CCRF-CEM |
|---|---|---|
| 4-Cl | > 4 μM, 25% inhibition at this concentration | >4 μM, 20% inhibition at this concentration |
| 3-$CH_3$ | >5 μM, 30% inhibition at this concentration | >5 μM, 20% inhibition at this concentration |
| 3-$CF_3$ | >4.3 μM, 35% inhibition at this concentration | >4.3 μM, 25% inhibition at this concentration |
| - | >10 μM, 35% inhibition at this concentration | >10 μM, 25% inhibition at this concentration |
| 3-$C_2H_5$ | >5 μM, 25% inhibition at this concentration | >5 μM, 30% inhibition at this concentration |
| 3-Br | >5.5 μM, 40% inhibition at this concentration | >5.5 μM, 25% inhibition at this concentration |
| 3-$CH_2OH$ | 48 μM[a] | >55 μM, 30% inhibition at this concentration |
| 3-CH(OH)$CH_3$ | >50 μM, 35% inhibition at this concentration | >50 μM, 35% inhibition at this concentration |
| 4-CN | >5 μM, 35% inhibition at this concentration | >5 μM, 10% inhibition at this concentration |
| 3-I | >5.3 μM, 35% inhibition at this concentration | >5.3 μM, 10% inhibition at this concentration |
| 3-Cl,4-CN | 4.5 μM[b] | >6.8 μM, 10% inhibition at this concentration |
| 4-$NO_2$ | 4.5 μM[c] | >4.8 μM, 10% inhibition at this concentration |
| Compound C | N.D[d] | N.D[d] |
| 3-C≡CH | >2.4 μM, 5% inhibition at this concentration | >2.4 μM, 15% inhibition at this concentration |
| Compound E | N.D | N.D |
| 3-$CF_3$,4-$NO_2$ | >5 μM, no inhibition at this concentration | N.D |
| Compound D | N.D | N.D |
| 4-COOH | 6.8 μM[e] | >8.5 μM, 40% inhibition at this concentration |

| | | |
|---|---|---|
| 3-$CF_3$,4-CN | > 2.8 μM, 15% inhibition at this concentration | > 2.8 μM, no inhibition at this concentration |
| 4-$SO_2$Ph | 1.0 μM | 2.5 μM |
| Compound A | > 2.75 μM, 45% inhibition at this concentration | > 2.75 μM, no inhibition at this concentration |
| 4-$SO_2NH_2$ | 20 μM[f] | >25 μM, no inhibition at this concentration |
| Compound B | 3.0 μM | 6.0 μM |
| 3 $CF_3$4-$SO_2NH_2$ | N.D | N.D |
| 3 $CF_3$4-$SO_2$NH -$CH_2$COOH | N.D | N.D |

[a]Tested to 55 μM, inhibition 55% at this concentration.
[b]Tested to 7 μM, inhibition 80% at this concentration.
[c]Tested to 4.7 μM, inhibition 55% at this concentration.
[d]insoluble at 1 mM in DMSO.
[e]Tested to 7.6 μm, inhibition 55% at this concentration.
[f]Tested to 26 μm, inhibition 60% at this concentration.
N.D - Not Determined

## Claims

1. A compound capable of inhibiting thymidylate synthase having the formula (I):

(I) $$R_L-CH_2-\underset{\displaystyle |}{\overset{R^2}{N}}-(RiSys)$$

wherein, in said formula (I):
$R_L$ is (i) a substituted or unsubstituted heterocyclic ring system or (ii) a group of the formula (II):

(II) 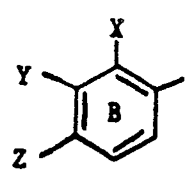

wherein, in said formula (II),
(a) X, Y and Z are independently hydrogen or separate substituents, with the proviso that at least one of X, Y and Z is not hydrogen,
(b) X and Y together comprise the nonmetallic atoms necessary to complete a substituted or unsubstituted homocyclic or heterocyclic ring, thereby forming a bicyclic ring system with phenyl group B, and Z is hydrogen or a separate substituent,
(c) Y and Z together comprise the nonmetallic atoms necessary to complete a substituted or unsubstituted, monocyclic or bicylic, homocyclic or heterocyclic ring system, thereby forming a bicyclic or tricyclic ring system with phenyl group B, and X is hydrogen or a separate substituent, with the proviso that when a 6-quinazolino group is so formed and X is a methyl group, the ring system does not have $-NH_2$ groups in

both the 2-and 4-positions, or

(d) X and Y together comprise the nonmetallic atoms necessary to complete a substituted or unsubstituted homocyclic or heterocyclic ring, and Y and Z together comprise the nonmetallic atoms necessary to complete a substituted or unsubstituted, nonocyclic or bicyclic, homocyclic or heterocyclic ring system, thereby forming a peri-fused tricyclic or tetracyclic ring system with phenyl group B;

with the proviso that $R_L$ is not a pteridine moiety, and where X is a separate substituent, X is selected from the group consisting of lower alkyl, lower haloalkyl, lower hydroxyalkyl, lower aminoalkyl, hydroxy, lower alkoxy, lower acyloxy, mercapto, lower alkylthio, lower alkylsulfinyl, lower alkylsulfonyl, lower acylthio, amino, lower alkylamino, lower acylamino, lower alkoxycarbonyl, lower acyl, carbamoyl, N-lower alkylcarbamoyl, halo, cyano, nitro and azido groups,

where Y is a separate substituent, Y is selected from the group consisting of alkyl, homoaryl, heteroaryl, hydroxyalkyl, aminoalkyl, alkylaminoalkyl, haloalkyl, hydroxy, alkoxy, aryloxy, acyloxy, aroyloxy, mercapto, alkylthio, alkylsulfinyl, alkylsulfonyl, arylthio, arylsulfinyl, arkylsulfonyl, pentafluorothio, acylthio, aroylthio, sulfamoyl, N-alkylsulfamoyl, N-arylsulfamoyl, sulfinamoyl, N-alkylsulfinamoyl, N-arylsulfinamoyl, N-hydroxysulfamyl, amino, alkylamino, arylamino, acylamino, aroylamino, sulfonamido, guanidino, hydrazino, acylhydrazino, alkoxycarbonyl, carbamoyl, N-alkylcarbamoyl, N-arylcarbamoyl, amidino, hydrazinocarbonyl, hydroxyaminocarbonyl, alkoxyaminocarbonyl, acyl, aroyl, halo, cyano, nitro, and azido groups, with alkyl and acyl groups containing from 1 to 6 carbon atoms, and

where Z is a separate substituent, Z is selected from the group consisting of those compounds defined above for substituent Y;

$R^2$ is hydrogen; substituted or unsubstituted alkyl having from 1 to 6 carbon atoms; substituted or unsubstituted alkenyl having from 1 to 6 carbon atoms; substituted or unsubstituted alkynyl having from 1 to 6 carbon atoms; aroylalkyl having from 1 to 10 carbon atoms; acyl having from 1 to 6 carbon atoms; $-OR'$ or $-N(R'R'')$ where $R'$ and $R''$ are independently hydrogen, alkyl, aryl, heteroaryl, acyl, aroyl, sulfonyl, sulfinyl or heteroaroyl groups; or $-SO_2-R'''$ or $-SO-R'''$ where $R'''$ is substituted or unsubstituted alkyl, aryl, heteroaryl or alkenyl; and

(RiSys) is a substituted or unsubstituted homocyclic or heterocyclic ring system having from 3 to 10 ring atoms,

with the following further provisos:

(i) when (RiSys) is substituted phenyl, (RiSys) is not substituted in the para-position with any of the following groups: $-SO_2$-glutamate; $-SO_2$-aspartate; or $-CO-NHR_b$ where $R_b$ is such that $NH_2-R_b$ is an amino acid, a poly(amino acid), a lower alkyl ester of an amino acid, or a lower alkyl ester of a poly(amino acid) and

(ii) when $R_L$ is 2-amino-3,4-dihydro-4-oxo-6-quinazolinyl and (RiSys) is substituted phenyl, (RiSys) is not substituted in the para-position with $-COOH$, $-(CH_2)_3-COOCH_3$, $-(CH_2)_3-COOH$, or $-COOC_2H_5$.

2. The compound of claim 1, wherein said compound has a thymidylate synthase inhibition constant $K_i$ of less than or equal to about $10^{-4}$ M.

3. The compound of claim 2, wherein said $K_i$ value is less than or equal to about $10^{-6}$ M.

4. The compound of claim 3, wherein said $K_i$ value is less than or equal to about $10^{-9}$ M.

5. The compound of claim 1, wherein, in formula (II), Z is hydrogen and X and Y together comprise the nometallic atoms necessary to complete a substituted or unsubstituted 5-membered or 6-membered heterocyclic ring, thereby forming a bicyclic ring system with phenyl group B, or

X is hydrogen and Y and Z together comprise the nonmetallic atoms necessary to complete a substituted or unsubstituted 5-membered or 6-membered heterocyclic ring, thereby forming a bicyclic ring system with phenyl group B, with the proviso that when the bicyclic ring system formed by Y and Z and phenyl group B is a 6-quinazolino group and X is a methyl group, the ring system does not comprise $-NH_2$ groups in both the 2- and 4-positions.

6. The compound of claim 5, wherein $R_L$ is a group of formula (II) having the structure

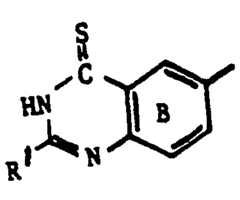

or

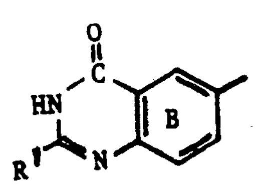

wherein $R^1$ is alkyl, cycloalkyl, alkenyl, alkynyl, alkoxy, alkylthio, aryl, aryloxy, arylalkyl, halogeno, hydroxy,

amino, mercapto, pyridylthio, pyrimidinylthio, substituted alkyl or substituted alkoxy.

7. The compound of claim 5, wherein $R_L$ is a group of formula (II) having the structure

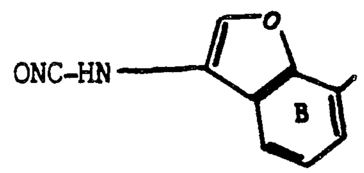

wherein said structure may be further substituted.

8. The compound of claim 1, wherein $R_L$ is a substituted or unsubstituted heterocyclic ring.

9. The compound of claim 8, wherein said heterocyclic ring is a thienopyrimidine ring.

10. The compound of claim 1, wherein $R^2$ is hydrogen, alkyl, alkenyl, alkynyl, halogenoalkyl, hydroxyalkyl, alkoxyalkyl, mercaptoalkyl or cyanoalkyl.

11. The compound of claim 10, wherein $R^2$ is hydrogen, methyl, ethyl, propyl, prop-2-enyl, prop-2-ynyl, cyanomethyl, 2-fluoroethyl, 2-hydroxyethyl, 2-methoxyethyl, 2-mercaptoethyl or 3-hydroxypropyl.

12. The compound of claim 1, wherein (RiSys) is a substituted or unsubstituted phenyl, naphthyl or heterocyclyl ring system.

13. The compound of claim 12, wherein (RiSys) is phenyl, thienyl or pyridyl.

14. The compound of claim 1, wherein (RiSys) is substituted with at least one electron donating or electron withdrawing group.

15. The compound of claim 13, wherein (RiSys) is substituted with a substituent $R^3$ and is also substituted with a substituent $R^4$, wherein

$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and

$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato or azido; or dialkylphosphinoyl, diarylphosphinoyl, dialkoxyphosphoryl, diaryloxyphosphoryl, dialkylaminophosphoryl or diarylaminophosphoryl.

16. The compound of claim 15, wherein (RiSys) is phenyl having the structure

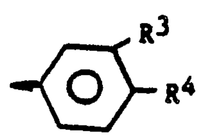

17. A compound capable of inhibiting thymidylate synthase having the formula (III):

(III) 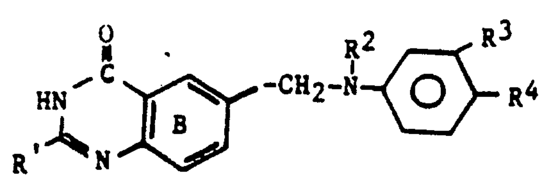 .

wherein:

$R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy;

$R^2$ is hydrogen, alkyl, alkenyl, alkynyl, halogenoalkyl, hydroxyalkyl, alkoxyalkyl, mercaptoalkyl or cyanoalkyl;

$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and

$R^4$ is hydrogen; substituted or unsubstituted alkyl, alkenyl, or alkynyl, each having from 1 to 6 carbons;

substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato or azido; or dialkylphosphinoyl, diarylphosphinoyl, dialkoxyphosphoryl, diaryloxyphosphoryl, dialkylaminophosphoryl or diarylaminophosphoryl.

18. The compound of claim 17 wherein $R^1$ is alkyl or alkoxy.

19. The compound of claim 18 wherein $R^1$ is methyl or methoxy.

20. The compound of claim 17, wherein $R^2$ is hydrogen, methyl, ethyl, propyl, prop-2-enyl, prop-2-ynyl, cyanomethyl, 2-fluoroethyl, 2-hydroxyethyl, 2-methoxyethyl, 2-mercaptoethyl or 3-hydroxypropyl.

21. The compound of claim 20 wherein $R^2$ is prop-2-ynyl.

22. The compound of claim 17 wherein $R^3$ is hydrogen; alkyl or alkynyl, each having from 1 to 6 carbon atoms; halogeno; cyano; azido; or substituted alkyl having from 1 to 3 carbon atoms.

23. The compound of claim 22 wherein $R^3$ is hydrogen, methyl, ethyl, ethynyl, trifluoromethyl, hydroxymethyl, alpha-hydroxyethyl, chloro, bromo, or iodo.

24. The compound of claim 17 wherein $R^4$ is hydrogen; substituted or unsubstituted alkylsulfonyl; substituted or unsubstituted aroyl; substituted or unsubstituted arylsulfonyl; nitro; sulfamoyl; pentafluorothio; (((carboxy)methyl)amino)sulfonyl; halogeno or cyano.

25. The compound of claim 24 wherein $R^4$ is hydrogen, fluoro, chloro, cyano, nitro, benzoyl, phenylsulfonyl, trifluoromethylsulfonyl, sulfamoyl, (((carboxy)methyl)amino)sulfonyl or pentafluorothio.

26. A compound capable of inhibiting thymidylate synthase having the formula (IV):

(IV) 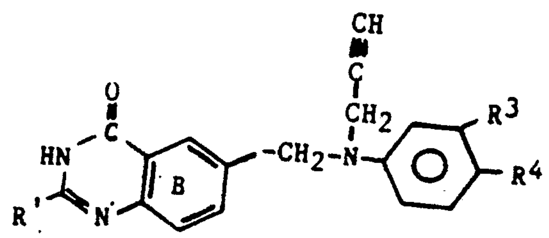

wherein:

$R^1$ is methyl, phenyl or methoxy;

$R^3$ is hydrogen, methyl, ethyl, ethynyl, trifluoromethyl, hydroxymethyl, alpha-hydroxyethyl, chloro, bromo, or iodo; and

$R^4$ is hydrogen, fluoro, chloro, cyano, nitro, benzoyl, phenylsulfonyl, trifluoromethylsulfonyl, sulfamoyl, ((-(carboxy)methyl)amino)sulfonyl or pentafluorothio.

27. The compound of claim 26 wherein $R^1$ is methyl, $R^3$ is hydrogen and $R^4$ is chloro.

28. The compound of claim 26 wherein $R^1$ is methyl, $R^3$ is methyl and $R^4$ is hydrogen.

29. The compound of claim 26 wherein $R^1$ is methyl, $R^3$ is trifluoromethyl and $R^4$ is hydrogen.

30. The compound of claim 26 wherein $R^1$ is methyl, $R^3$ is ethyl and $R^4$ is hydrogen.

31. The compound of claim 26 wherein $R^1$ is methyl, $R^3$ is bromo and $R^4$ is hydrogen.

32. The compound of claim 26 wherein $R^1$ is methyl, $R^3$ is hydroxymethyl and $R^4$ is hydrogen.

33. The compound of claim 26 wherein $R^1$ is methyl, $R^3$ is alpha-hydroxyethyl and $R^4$ is hydrogen.

34. The compound of claim 26 wherein $R^1$ is methyl, $R^3$ is hydrogen and $R^4$ is cyano.

35. The compound of claim 26 wherein $R^1$ is methyl, $R^3$ is iodo and $R^4$ is hydrogen.

36. The compound of claim 26 wherein $R^1$ is methyl, $R^3$ is chloro and $R^4$ is cyano.

37. The compound of claim 26 wherein $R^1$ is methyl, $R^3$ is hydrogen and $R^4$ is nitro.

38. The compound of claim 26 wherein $R^1$ is methyl, $R^3$ is ethynyl and $R^4$ is hydrogen.

39. The compound of claim 26 wherein $R^1$ is methyl, $R^3$ is trifluoromethyl and $R^4$ is nitro.

40. The compound of claim 26 wherein $R^1$ is methyl, $R^3$ is hydrogen and $R^4$ is benzoyl.

41. The compound of claim 26 wherein $R^1$ is methyl, $R^3$ is trifluoromethyl and $R^4$ is cyano.

42. The compound of claim 26 wherein $R^1$ is methyl, $R^3$ is hydrogen and $R^4$ is phenylsulfonyl.

43. The compound of claim 26 wherein $R^1$ is methyl, $R^3$ is hydrogen and $R^4$ is trifluoromethylsulfonyl.

44. The compound of claim 26 wherein $R^1$ is methyl, $R^3$ is hydrogen and $R^4$ is sulfamoyl.

45. The compound of claim 26 wherein $R^1$ is methyl, $R^3$ is trifluoromethyl and $R^4$ is sulfamoyl.

46. The compound of claim 26 wherein $R^1$ is methyl, $R^3$ is trifluoromethyl and $R^4$ is $-SO_2NHCH_2COOH$.

47. The compound of claim 26 wherein $R^1$ is methyl, $R^3$ is trifluoromethyl and $R^4$ is phenylsulfonyl.

48. The compound of claim 26 wherein $R^1$ is phenyl, $R^3$ is hydrogen and $R^4$ is chloro.

49. The compound of claim 6, wherein $R^2$ is prop-2-ynyl and (RiSys) is 3-trifluoromethylphenyl.

50. The compound of claim 7, wherein $R^2$ is prop-2-ynyl and (RiSys) is 4-nitrophenyl.

51. A process for making a compound of the formula (I)

(I) 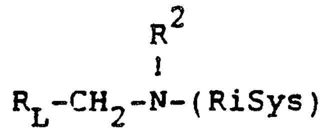

wherein, in said formula (I):

$R_L$ is (i) a substituted or unsubstituted heterocyclic ring or (ii) a group of the formula (II):

(II) 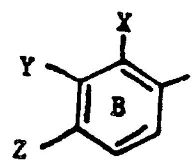

wherein, in said formula (II),

(a) X, Y and Z are independently hydrogen or separate substituents, with the proviso that at least one of X, Y and Z is not hydrogen,

(b) X and Y together comprise the nonmetallic atoms necessary to complete a substituted or unsubstituted homocyclic or heterocyclic ring, thereby forming a bicyclic ring system with phenyl group B, and Z is hydrogen or a separate substituent,

(c) Y and Z together comprise the nonmetallic atoms necessary to complete a substituted or unsubstituted, monocyclic or bicylic, homocyclic or heterocyclic ring system, thereby forming a bicyclic or tricyclic ring system with phenyl group B, and X is hydrogen or a separate substituent, with the proviso that when a 6-quinazolino group is so formed and X is a methyl group, the ring system does not have $-NH_2$ groups in both the 2-and 4-positions, or

(d) X and Y together comprise the nonmetallic atoms necessary to complete a substituted or unsubstituted homocyclic or heterocyclic ring, and Y and Z together comprise the nonmetallic atoms necessary to complete a substituted or unsubstituted, nonocyclic or bicyclic, homocyclic or heterocyclic ring system, thereby forming a peri-fused tricyclic or tetracyclic ring system with phenyl group B;

with the proviso that $R_L$ is not a pteridine moiety, and where X is a separate substituent, X is selected from the group consisting of lower alkyl, lower haloalkyl, lower hydroxyalkyl, lower aminoalkyl, hydroxy, lower alkoxy, lower acyloxy, mercapto, lower alkylthio, lower alkylsulfinyl, lower alkylsulfonyl, lower acylthio, amino, lower alkylamino, lower acylamino, lower alkoxycarbonyl, lower acyl, carbamoyl, N-lower alkylcarbamoyl, halo, cyano, nitro and azido groups,

where Y is a separate substituent, Y is selected from the group consisting of alkyl, homoaryl, heteroaryl, hydroxyalkyl, aminoalkyl, alkylaminoalkyl, haloalkyl, hydroxy, alkoxy, aryloxy, acyloxy, aroyloxy, mercapto, alkylthio, alkylsulfinyl, alkylsulfonyl, arylthio, arylsulfinyl, arkylsulfonyl, pentafluorothio, acylthio, aroylthio, sulfamoyl, N-alkylsulfamoyl, N-arylsulfamoyl, sulfinamoyl, N-alkylsulfinamoyl, N-arylsulfinamoyl, N-hydroxysulfamyl, amino, alkylamino, arylamino, acylamino, aroylamino, sulfonamido, guanidino, hydrazino, acylhydrazino, alkoxycarbonyl, carbamoyl, N-alkylcarbamoyl, N-arylcarbamoyl, amidino, hydrazinocarbonyl, hydroxyaminocarbonyl, alkoxyaminocarbonyl, acyl, aroyl, halo, cyano, nitro, and azido groups, with alkyl and acyl groups containing from 1 to 6 carbon atoms, and

where Z is a separate substituent, Z is selected from the group consisting of those compounds defined above for substituent Y;

$R^2$ is hydrogen; substituted or unsubstituted alkyl having from 1 to 6 carbon atoms; substituted or unsubstituted alkenyl having from 1 to 6 carbon atoms; substituted or unsubstituted alkynyl having from 1 to 6 carbon atoms; aroylalkyl having from 1 to 10 carbon atoms; acyl having from 1 to 6 carbon atoms; $-OR'$ or $-N(R'R'')$ where $R'$ and $R''$ are independently hydrogen, alkyl, aryl, heteroaryl, acyl, aroyl, sulfonyl, sulfinyl or heteroaroyl groups; or $-SO_2-R'''$ or $-SO-R'''$ where $R'''$ is substituted or unsubstituted alkyl, aryl, heteroaryl or alkenyl; and

(RiSys) is a substituted or unsubstituted homocyclic or heterocyclic ring system having from 3 to 10 ring atoms,

with the following further provisos:

(i) when (RiSys) is substituted phenyl, (RiSys) is not substituted in the para-position with any of the following groups: $-SO_2$-glutamate; $-SO_2$-aspartate; or $-CO-NHR_b$ where $R_b$ is such that $NH_2-R_b$ is an amino acid, a poly(amino acid), a lower alkyl ester of an amino acid, or a lower alkyl ester of a poly(amino acid) and

(ii) when $R_L$ is 2-amino-3,4-dihydro-4-oxo-6-quinazolinyl and (RiSys) is substituted phenyl, (RiSys) is not substituted in the para-position with -COOH, $-(CH_2)_3-COOCH_3$, $-(CH_2)_3-COOH$, or $-COOC_2H_5$,

comprising the steps of:

(1) allowing a compound of the formula $R_L-CH_2-D$ to react with a compound of the formula

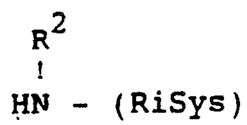

wherein D is a displaceable group, provided that one or more of $R_L$, $R^2$ and (RiSys) may contain a chemical group or groups which may be protected by a protecting group during the course of the reaction; and

(2) selectively removing one or more of any protecting groups in $R_L$, $R^2$ and (RiSys).

52. The process of claim 51 wherein the compound of formula (I) has a thymidylate synthase inhibition constant $K_i$ of less than or equal to about $10^{-4}$M.

53. The process of claim 51 wherein $R_L$ is a group of formula (II) having the structure

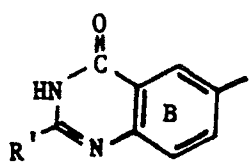

or

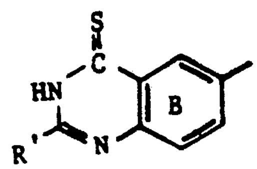

wherein $R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy.

54. The process of claim 51 wherein $R^2$ is hydrogen, alkyl, alkenyl, alkynyl, halogenoalkyl, hydroxyalkyl, alkoxyalkyl, mercaptoalkyl or cyanoalkyl.

55. The process of claim 51 wherein (RiSys) is a substituted or unsubstituted phenyl, naphthyl or heterocyclyl ring system.

56. The process of claim 51 wherein (RySys) is phenyl having the structure

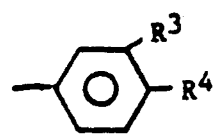

wherein:

$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and

$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato or azido; or dialkylphosphinoyl, diarylphosphinoyl, dialkoxyphosphoryl, diaryloxyphosphoryl, dialkylaminophosphoryl or diarylaminophosphoryl.

57. The process of claim 51 wherein the compound of formula (I) has the formula (III)

(III) 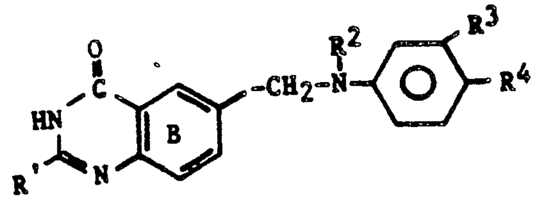

wherein:
$R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy;
$R^2$ is hydrogen, alkyl, alkenyl, alkynyl, halogenoalkyl, hydroxyalkyl, alkoxyalkyl, mercaptoalkyl or cyanoalkyl;
$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and
$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato or azido; or dialkylphosphinoyl, diarylphosphinoyl, dialkoxyphosphoryl, diaryloxyphosphoryl, dialkylaminophosphoryl or diarylaminophosphoryl.

58. The process of claim 57 wherein:
$R^1$ is methyl or methoxy;
$R^2$ is prop-2-ynyl;
$R^3$ is hydrogen, methyl, ethyl, ethynyl, trifluoromethyl, hydroxymethyl, alpha-hydroxyethyl, chloro, bromo, or iodo; and
$R^4$ is hydrogen, fluoro, chloro, cyano, nitro, benzoyl, phenylsulfonyl, trifluoromethylsulfonyl, sulfamoyl, ((-(carboxy)methyl)amino)sulfonyl or pentafluorothio.

59. The process of claim 51 wherein D is chloro, bromo or a substituted sulfonyloxy group.

60. The process of claim 59, wherein D is a methanesulfonyloxy, trifluoromethanesulfonyloxy, toluene-p-sulfonyloxy or 4-bromobenzenesulfonyloxy group.

61. The process of claim 51 wherein said one or more protecting groups is selected from the group consisting of an esterifying group, an alpha-arylalkyl group, a substituted or unsubstituted alkoxycarbonyl group, a phthaloyl group, or methyl oxyether-type group or a pivaloyloxymethyl group.

62. The process of claim 51 wherein the compound of the formula

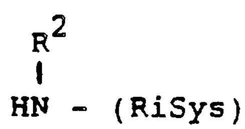

is prepared by the reaction of the bromide, chloride or tosylate of $R^2$ with a compound of formula (V),
(V) $H_2N$ - (RiSys)

63. The process of claim 62 wherein $R^2$ is prop-2-ynyl and the tosylate of $R^2$ is reacted with a compound of formula (V).

64. The process of claim 51, wherein the compound $CF_3$-CONH-(RiSys) is treated with a base to form a first intermediate compound, said first intermediate compound is then contacted with the compound $R^2$-D to form a second intermediate compound, and wherein said second intermediate compound is hydrolyzed to form the compound

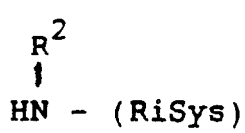

65. The process of claim 64, wherein the base is sodium hydride.

66. The process of claim 51, wherein the compound D-(RiSys), the chemical nature of the (RiSys) group being such as to activate the D group, is contacted with the compound $R^2$-$NH_2$ to form the compound

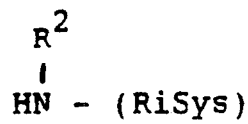

67. The process of claim 51 wherein the compound

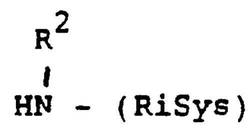

is treated with a base prior to being brought into contact with the compound $R_L$-$CH_2$-D.

68. The process of claim 67 wherein the base is sodium hydride.

69. A pharmaceutical composition comprising a pharmaceutically acceptable diluent or carrier in combination with an amount effective to inhibit thymidylate synthase of a compound having the formula:

(I) 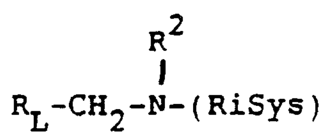

wherein, in said formula (I):

$R_L$ is (i) a substituted or unsubstituted heterocyclic ring system or (ii) a group of the formula (II):

(II) 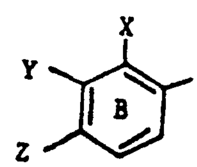

wherein, in said formula (II),

(a) X, Y and Z are independently hydrogen or separate substituents, with the proviso that at least one of X, Y and Z is not hydrogen,

(b) X and Y together comprise the nonmetallic atoms necessary to complete a substituted or unsubstituted homocyclic or heterocyclic ring, thereby forming a bicyclic ring system with phenyl group B, and Z is hydrogen or a separate substituent,

(c) Y and Z together comprise the nonmetallic atoms necessary to complete a substituted or unsubstituted, monocyclic or bicylic, homocyclic or heterocyclic ring system, thereby forming a bicyclic or tricyclic ring system with phenyl group B, and X is hydrogen or a separate substituent, with the proviso that when a 6-quinazolino group is so formed and X is a methyl group, the ring system does not have -$NH_2$ groups in both the 2-and 4-positions, or

(d) X and Y together comprise the nonmetallic atoms necessary to complete a substituted or unsubstituted homocyclic or heterocyclic ring, and Y and Z together comprise the nonmetallic atoms necessary to complete a substituted or unsubstituted, nonocyclic or bicyclic, homocyclic or heterocyclic ring system, thereby forming a peri-fused tricyclic or tetracyclic ring system with phenyl group B;

with the proviso that $R_L$ is not a pteridine moiety, and where X is a separate substituent, X is selected from the group consisting of lower alkyl, lower haloalkyl, lower hydroxyalkyl, lower aminoalkyl, hydroxy, lower alkoxy, lower acyloxy, mercapto, lower alkylthio, lower alkylsulfinyl, lower alkylsulfonyl, lower acylthio, amino, lower alkylamino, lower acylamino, lower alkoxycarbonyl, lower acyl, carbamoyl, N-lower alkylcarbamoyl, halo, cyano, nitro and azido groups,

where Y is a separate substituent, Y is selected from the group consisting of alkyl, homoaryl, heteroaryl, hydroxyalkyl, aminoalkyl, alkylaminoalkyl, haloalkyl, hydroxy, alkoxy, aryloxy, acyloxy, aroyloxy, mercapto, alkylthio, alkylsulfinyl, alkylsulfonyl, arylthio, arylsulfinyl, arkylsulfonyl, pentafluorothio, acylthio, aroylthio, sulfamoyl, N-alkylsulfamoyl, N-arylsulfamoyl, sulfinamoyl, N-alkylsulfinamoyl, N-arylsulfinamoyl, N-hydroxysulfamyl, amino, alkylamino, arylamino, acylamino, aroylamino, sulfonamido, guanidino, hydrazino, acylhydrazino, alkoxycarbonyl, carbamoyl, N-alkylcarbamoyl, N-arylcarbamoyl, amidino, hydrazinocarbonyl, hydroxyaminocarbonyl, alkoxyaminocarbonyl, acyl, aroyl, halo, cyano, nitro, and azido groups, with alkyl and acyl groups containing from 1 to 6 carbon atoms, and

where Z is a separate substituent, Z is selected from the group consisting of those compounds defined above for substituent Y;

$R^2$ is hydrogen; substituted or unsubstituted alkyl having from 1 to 6 carbon atoms; substituted or unsubstituted alkenyl having from 1 to 6 carbon atoms; substituted or unsubstituted alkynyl having from 1 to 6 carbon atoms; aroylalkyl having from 1 to 10 carbon atoms; acyl having from 1 to 6 carbon atoms; $-OR'$ or $-N(R'R'')$ where $R'$ and $R''$ are independently hydrogen, alkyl, aryl, heteroaryl, acyl, aroyl, sulfonyl, sulfinyl or heteroaroyl groups; or $-SO_2-R'''$ or $-SO-R'''$ where $R'''$ is substituted or unsubstituted alkyl, aryl, heteroaryl or alkenyl; and

(RiSys) is a substituted or unsubstituted homocyclic or heterocyclic ring system having from 3 to 10 ring atoms,

with the following further provisos:

(i) when (RiSys) is substituted phenyl, (RiSys) is not substituted in the para-position with any of the following groups: $-SO_2$-glutamate; $-SO_2$-aspartate; or $-CO-NHR_b$ where $R_b$ is such that $NH_2-R_b$ is an amino acid, a poly(amino acid), a lower alkyl ester of an amino acid, or a lower alkyl ester of a poly(amino acid) and

(ii) when $R_L$ is 2-amino-3,4-dihydro-4-oxo-6-quinazolinyl and (RiSys) is substituted phenyl, (RiSys) is not substituted in the para-position with -COOH, $-(CH_2)_3-COOCH_3$, $-(CH_2)_3-COOH$, or $-COOC_2H_5$.

70. The composition of claim 69 wherein said amount is a nontoxic amount.

71. The composition of claim 69 wherein the compound of formula (I) has a thymidylate synthase inhibition constant $K_i$ of less than or equal to about $10^{-4}$M.

72. The composition of claim 69 wherein $R_L$ is a group of formula (II) having the structure

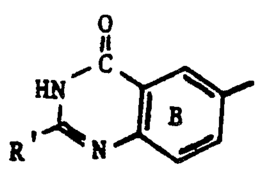

or

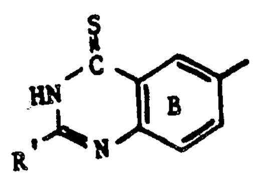

wherein $R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy.

73. The composition of claim 69 wherein $R^2$ is hydrogen, alkyl, alkenyl, alkynyl, halogenoalkyl, hydroxyalkyl, alkoxyalkyl, mercaptoalkyl or cyanoalkyl.

74. The composition of claim 69 wherein (RiSys) is a substituted or unsubstituted phenyl, naphthyl or heterocyclyl ring system.

75. The composition of claim 69 wherein (RiSys) is phenyl having the structure

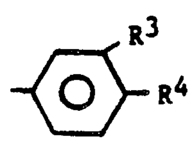

wherein:

$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and

$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl,

arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato, or azido; or dialkylphosphinoyl, diarylphosphinoyl, dialkoxyphosphoryl, diaryloxyphosphoryl, dialkylaminophosphoryl or diarylaminophosphoryl.

76. The composition of claim 69 wherein the compound of formula (I) has the formula (III)

(III) 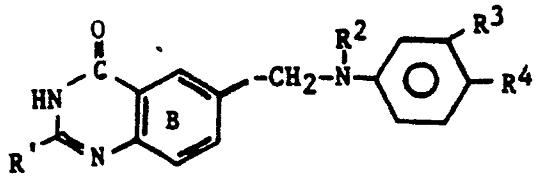

wherein:
$R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy;
$R^2$ is hydrogen, alkyl, alkenyl, alkynyl, halogenoalkyl, hydroxyalkyl, alkoxyalkyl, mercaptoalkyl or cyanoalkyl;
$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and
$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato, or azido; or dialkylphosphinoyl, diarylphosphinoyl, dialkoxyphosphoryl, diaryloxyphosphoryl, dialkylaminophosphoryl or diarylaminophosphoryl.

77. The composition of claim 76 wherein:
$R^1$ is methyl or methoxy;
$R^2$ is prop-2-ynyl;
$R^3$ is hydrogen, methyl, ethyl, ethynyl, trifluoromethyl, hydroxymethyl, amino, alpha-hydroxyethyl, chloro, bromo, or iodo; and
$R^4$ is hydrogen, fluoro, chloro, cyano, nitro, benzoyl, phenylsulfonyl, trifluoromethylsulfonyl, sulfamoyl, ((-(carboxy)methyl)amino)sulfonyl or pentafluorothio.

78. The composition of claim 69 in a form selected from the group consisting of forms suitable for oral, parenteral, topical, intravaginal, intranasal, intrabronchial, intraocular, intraaural and rectal administration.

79. The composition of claim 69 further comprising at least one other compound which is an antitumor agent.

80. The composition of claim 79 wherein said at least one other compound is selected from the group consisting of mitotic inhibitors, alkylating agents, DHFR inhibitors, antimetabolites, intercalating antibiotics, enzymes, topoisomerase inhibitors or biological response modifiers.

81. The composition of claim 69 further comprising at least one other compound which is an antibacterial agent, an antifungal agent, an antiparasitic agent, an antiviral agent, an antipsoriatic agent, an antiprotozoal agent or an anticoccidial agent.

82. A therapeutic process of inhibiting thymidylate synthase comprising administering to a vertebrate host an amount effective to inhibit thymidylate synthase of a compound of the formula:

(I) 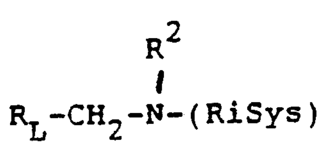

wherein, in said formula (I):
$R_L$ is (i) a substituted or unsubstituted heterocyclic ring system or (ii) a group of the formula (II):

(II) 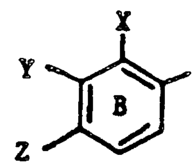

wherein, in said formula (II),

(a) X, Y and Z are independently hydrogen or separate substituents, with the proviso that at least one of X, Y and Z is not hydrogen,

(b) X and Y together comprise the nonmetallic atoms necessary to complete a substituted or unsubstituted homocyclic or heterocyclic ring, thereby forming a bicyclic ring system with phenyl group B, and Z is hydrogen or a separate substituent,

(c) Y and Z together comprise the nonmetallic atoms necessary to complete a substituted or unsubstituted, monocyclic or bicylic, homocyclic or heterocyclic ring system, thereby forming a bicyclic or tricyclic ring system with phenyl group B, and X is hydrogen or a separate substituent, with the proviso that when a 6-quinazolino group is so formed and X is a methyl group, the ring system does not have $-NH_2$ groups in both the 2-and 4-positions, or

(d) X and Y together comprise the nonmetallic atoms necessary to complete a substituted or unsubstituted homocyclic or heterocyclic ring, and Y and Z together comprise the nonmetallic atoms necessary to complete a substituted or unsubstituted, nonocyclic or bicyclic, homocyclic or heterocyclic ring system, thereby forming a peri-fused tricyclic or tetracyclic ring system with phenyl group B;

with the proviso that $R_L$ is not a pteridine moiety, and where X is a separate substituent, X is selected from the group consisting of lower alkyl, lower haloalkyl, lower hydroxyalkyl, lower aminoalkyl, hydroxy, lower alkoxy, lower acyloxy, mercapto, lower alkylthio, lower alkylsulfinyl, lower alkylsulfonyl, lower acylthio, amino, lower alkylamino, lower acylamino, lower alkoxycarbonyl, lower acyl, carbamoyl, N-lower alkylcarbamoyl, halo, cyano, nitro and azido groups,

where Y is a separate substituent, Y is selected from the group consisting of alkyl, homoaryl, heteroaryl, hydroxyalkyl, aminoalkyl, alkylaminoalkyl, haloalkyl, hydroxy, alkoxy, aryloxy, acyloxy, aroyloxy, mercapto, alkylthio, alkylsulfinyl, alkylsulfonyl, arylthio, arylsulfinyl, arkylsulfonyl, pentafluorothio, acylthio, aroylthio, sulfamoyl, N-alkylsulfamoyl, N-arylsulfamoyl, sulfinamoyl, N-alkylsulfinamoyl, N-arylsulfinamoyl, N-hydroxysulfamyl, amino, alkylamino, arylamino, acylamino, aroylamino, sulfonamido, guanidino, hydrazino, acylhydrazino, alkoxycarbonyl, carbamoyl, N-alkylcarbamoyl, N-arylcarbamoyl, amidino, hydrazinocarbonyl, hydroxyaminocarbonyl, alkoxyaminocarbonyl, acyl, aroyl, halo, cyano, nitro, and azido groups, with alkyl and acyl groups containing from 1 to 6 carbon atoms, and

where Z is a separate substituent, Z is selected from the group consisting of those compounds defined above for substituent Y;

$R^2$ is hydrogen; substituted or unsubstituted alkyl having from 1 to 6 carbon atoms; substituted or unsubstituted alkenyl having from 1 to 6 carbon atoms; substituted or unsubstituted alkynyl having from 1 to 6 carbon atoms; aroylalkyl having from 1 to 10 carbon atoms; acyl having from 1 to 6 carbon atoms; $-OR'$ or $-N(R'R'')$ where $R'$ and $R''$ are independently hydrogen, alkyl, aryl, heteroaryl, acyl, aroyl, sulfonyl, sulfinyl or heteroaroyl groups; or $-SO_2-R'''$ or $-SO-R'''$ where $R'''$ is substituted or unsubstituted alkyl, aryl, heteroaryl or alkenyl; and

(RiSys) is a substituted or unsubstituted homocyclic or heterocyclic ring system having from 3 to 10 ring atoms,

with the following further provisos:

(i) when (RiSys) is substituted phenyl, (RiSys) is not substituted in the para-position with any of the following groups: $-SO_2$-glutamate; $-SO_2$-aspartate; or $-CO-NHR_b$ where $R_b$ is such that $NH_2-R_b$ is an amino acid, a poly(amino acid), a lower alkyl ester of an amino acid, or a lower alkyl ester of a poly(amino acid) and

(ii) when $R_L$ is 2-amino-3,4-dihydro-4-oxo-6-quinazolinyl and (RiSys) is substituted phenyl, (RiSys) is not substituted in the para-position with $-COOH$, $-(CH_2)_3-COOCH_3$, $-(CH_2)_3-COOH$, or $-COOC_2H_5$.

83. The process of claim 82 wherein the compound of formula (I) has a thymidylate synthase inhibition constant $K_i$ of less than or equal to about $10^{-4}$M.

84. The process of claim 82 wherein $R_L$ is a group of formula (II) having the structure

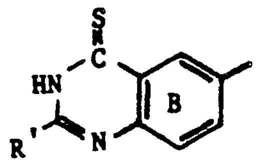 or 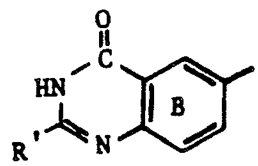

wherein $R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy.

85. The process of claim 82 wherein $R^2$ is hydrogen, alkyl, alkenyl, alkynyl, halogenoalkyl, hydroxyalkyl, alkoxyalkyl, mercaptoalkyl or cyanoalkyl.

86. The process of claim 82 wherein (RiSys) is a substituted or unsubstituted phenyl, naphthyl or heterocyclyl ring system.

87. The process of claim 82 wherein (RiSys) is phenyl having the structure

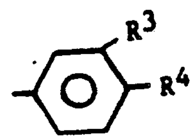

wherein:

$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and

$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato, or azido; or dialkylphosphinoyl, diarylphosphinoyl, dialkoxyphosphoryl, diaryloxyphosphoryl, dialkylaminophosphoryl or diarylaminophosphoryl.

88. The process of claim 82 wherein the compound of formula (I) has the formula (III)

(III) 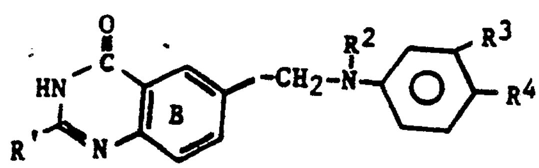

wherein:

$R^1$ is alkyl, cycloalkyl, alkenyl, alkynyl, alkoxy, alkylthio, aryl, aryloxy, arylalkyl, halogeno, hydroxy, amino, mercapto, pyridylthio, pyrimidinylthio, substituted alkyl or substituted alkoxy;

$R^2$ is hydrogen, alkyl, alkenyl, alkynyl, halogenoalkyl, hydroxyalkyl, alkoxyalkyl, mercaptoalkyl or cyanoalkyl;

$R^3$ is hydrogen; alkyl, alkenyl or alkynyl, each having from 1 to 6 carbon atoms; halogeno, cyano, cyanato, thiocyanato, selenocyanato, or azido; or substituted alkyl having from 1 to 3 carbon atoms; and

$R^4$ is hydrogen; substituted or unsubstituted alkyl, alkenyl, or alkynyl, each having from 1 to 6 carbons; substituted or unsubstituted alkylthio, alkylsulfinyl, alkylsulfonyl, alkenethio, alkenesulfinyl or alkenesulfonyl, each having from 1 to 6 carbon atoms; substituted or unsubstituted aroyl, heteroaroyl, arylthio, arylsulfinyl, arylsulfonyl, heteroarylthio, heteroarylsulfinyl, heteroarylsulfonyl; pentafluorothio; or nitro, sulfamoyl, ((-(carboxy)methyl)amino)sulfonyl, sulfinamoyl, halogeno, cyano, cyanato, thiocyanato, selenocyanato, or azido; or dialkylphosphinoyl, diarylphosphinoyl, dialkoxyphosphoryl, diaryloxyphosphoryl, dialkylaminophosphoryl or diarylaminophosphoryl.

89. The process of claim 88 wherein:

$R^1$ is methyl or methoxy;

$R^2$ is prop-2-ynyl;

$R^3$ is hydrogen, methyl, ethyl, ethynyl, trifluoromethyl, hydroxymethyl, alpha-hydroxyethyl, chloro, bromo, or iodo; and

$R^4$ is hydrogen, fluoro, chloro, cyano, nitro, benzoyl, phenylsulfonyl, trifluoromethylsulfonyl, sulfamoyl, ((-(carboxy)methyl)amino)sulfonyl or pentafluorothio.

90. The composition of claim 82 in a form selected from the group consisting of forms suitable for oral, parenteral, topical, intravaginal, intranasal, intrabronchial, intraocular, intraaural and rectal administration.

91. The process of claim 82 wherein the dose of the compound of formula (I) is up to about 5,000 mg per square meter of the body area of said vertebrate host.

92. The process of claim 82 wherein said vertebrate host is a mammal.

93. The process of claim 92 wherein said vertebrate host is human.

94. The process of claim 82 wherein said vertebrate host is a chicken.

95. The process of claim 82 wherein the compound of formula (I) is further characterized as producing an antiproliferative effect.

96. The process of claim 82 wherein, prior to said process, said vertebrate host harbors tumorous cells and wherein the compound of formula (I) is further characterized as producing an antitumor effect.

97. The process of claim 82 wherein the compound of formula (I) is further characterized as producing at least one of the following effects: antibacterial, antifungal, antiparasitic, antiviral, antipsoriatic, antiprotozoal or anticoccidial.

European Patent Office

# EUROPEAN SEARCH REPORT

Application Number

EP 89 11 3994

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 284 338 (IMPERIAL CHEMICAL INDUSTRIES) * page 17, claim 1; page 2, lines 1-15 * | 1,69 | C 07 D 239/84<br>C 07 D 239/88<br>C 07 D 239/90<br>C 07 D 239/91<br>C 07 D 239/93<br>C 07 D 239/95<br>C 07 D 307/89<br>C 07 D 401/12<br>C 07 D 405/12<br>C 07 D 417/12<br>A 61 K 31/505//<br>(C 07 D 401/12<br>C 07 D 239:00<br>C 07 D 213:00 )<br>(C 07 D 405/12<br>C 07 D 307:00<br>C 07 D 213:00 )<br>-/- |
| A,D | EP-A-0 239 362 (IMPERIAL CHEMICAL INDUSTRIES et al.) * pages 1-3 * | 1,69 | |
| A | EP-A-0 204 529 (NATIONAL RESEARCH DEVELOPMENT) * page 6, lines 3-27; pages 8-11 * | 1,69 | |
| A | EP-A-0 031 237 (NATIONAL RESEARCH DEVELOPMENT) * page 3, lines 15-25; pages 4-8 * | 1,69 | |
| A | JOURNAL OF MEDICINAL CHEMISTRY vol. 30, 1987, pages 675-678, Washington, USA; D.I. BRIXNER et al.: "Folate Analogues as Inhibitors of Thymidylate Synthase" * the whole article * | 1,69 | **TECHNICAL FIELDS SEARCHED (Int. Cl.5)** |
| A | JOURNAL OF MEDICINAL CHEMISTRY vol. 28, 1985, pages 1468-1476, Washington, USA; T.R. JONES et al.: "Quinazoline Antifolates Inhibiting Thymidylate Synthase: Variation of the N10 Substituent" * the whole article * | 1,69 | C 07 D 239/00<br>C 07 D 307/00<br>C 07 D 401/00<br>C 07 D 405/00<br>C 07 D 417/00 |

The present search report has been drawn up for all claims

| Place of search | Date of completion of the search | Examiner |
|---|---|---|
| BERLIN | 20-12-1989 | KYRIAKAKOU G |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 89 11 3994

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | | | (C 07 D 417/12<br>C 07 D 333:00<br>C 07 D 307:00 ) |
| | | | TECHNICAL FIELDS SEARCHED (Int. Cl.5) |
| | | | |

The present search report has been drawn up for all claims

| Place of search | Date of completion of the search | Examiner |
|---|---|---|
| BERLIN | 20-12-1989 | KYRIAKAKOU G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)